# EUROPEAN PATENT SPECIFICATION

(11) **EP 1 287 024 B1**
(45) Date of publication and mention of the grant of the patent: **26.12.2007**
(21) Application number: 01925742.7
(22) Date of filing: 08.05.2001
(51) Int. Cl.: C07K 14/195

(54) **VIRULENCE GENES, PROTEINS, AND THEIR USE**
VIRULENZ GENE, PROTEINE, UND IHRE VERWENDUNG
GENES DE VIRULENCE, PROTEINES, ET LEUR UTILISATION

(30) Priority: 08.05.2000 GB 0011108
(43) Date of publication of application: 05.03.2003
(60) Divisional application: 07114502.3
(73) Proprietor: Microscience Limited, Wokingham, Berkshire RG41 5TU (GB)
(72) Inventor: TANG, Christoph, Imperial College of Science, London SW7 2AZ (GB)
(74) Representative: Jappy, John William Graham
(86) International application number: PCT/GB2001/002003
(87) International publication number: WO 2001/085772

(56) References cited:
- WO-A-00/22430
- WO-A-00/66791
- WO-A-96/12020
- WO-A-98/02547
- WO-A-98/17805
- WO-A-98/56901
- WO-A-99/09176
- WO-A-99/24578
- WO-A-99/55875
- US-A- 6 015 669
- PARKHILL J ET AL: "COMPLETE DNA SEQUENCE OF A SEROGROUP A STRAIN OF NEISSERIA MENINGITIDIS Z2491" NATURE,MACMILLAN JOURNALS LTD. LONDON,GB, [Online] vol. 404, 30 March 2000 (2000-03-30), pages 502-506, XP000918875 ISSN: 0028-0836 -& DATABASE EMBL [Online] 30 March 2000 (2000-03-30) retrieved from EBI Database accession no. NMA3Z2491 XP002182574 -& DATABASE EMBL [Online] 30 March 2000 (2000-03-30) retrieved from EBI Database accession no. NMA5Z2491 XP002182575 -& DATABASE EMBL [Online] 30 March 2000 (2000-03-30) retrieved from EBI Database accession no. NMA6Z2491 XP002182576
- TETTELIN H ET AL: "COMPLETE GENOME SEQUENCE OF NEISSERIA MENINGITIDIS SEROGROUP B STRAIN MC58" SCIENCE, vol. 287, 10 March 2000 (2000-03-10), pages 1809-1815, XP000914963 ISSN: 0036-8075 & DATABASE EMBL [Online] 15 March 2000 (2000-03-15) retrieved from EBI Database accession no. AE002543 XP002182539 -& DATABASE EMBL [Online] 15 March 2000 (2000-03-15) retrieved from EBI Database accession no. AE002430 XP002182577 -& DATABASE EMBL [Online] 20 March 2000 (2000-03-20) retrieved from EBI Database accession no. AE002410 XP002182578 -& DATABASE EMBL [Online] 15 March 2000 (2000-03-15) retrieved from EBI Database accession no. AE002520 XP002182579 -& DATABASE EMBL [Online] 15 March 2000 (2000-03-15) retrieved from EBI Database accession no. AE002523 XP002182580 -& DATABASE EMBL [Online] 15 March 2000 (2000-03-15) retrieved from EBI Database accession no. AE002502 XP002182581
- REGNIER P ET AL: "NUCLEOTIDE SEQUENCE OF THE PNP GENE OF ESCHERICHIA-COLI ENCODING POLYNUCLEOTIDE PHOSPHORYLASE HOMOLOGY OF THE PRIMARY STRUCTURE OF THE PROTEIN WITH THE RNA-BINDING DOMAIN OF RIBOSOMAL PROTEIN S1" JOURNAL OF BIOLOGICAL CHEMISTRY, vol. 262, no. 1, 1987, pages 63-68, XP001022419 ISSN: 0021-9258 -& DATABASE EMBL [Online] 7 June 1987 (1987-06-07) retrieved from EBI Database accession no. J02638 XP002182582
- DATABASE SWALL [Online] 1 November 1997 (1997-11-01) retrieved from EBI Database accession no. Q57251 XP002182144
- DATABASE SWALL [Online] 1 August 1992 (1992-08-01) retrieved from EBI Database accession no. P26615 XP002182145
- STOJILJKOVIC IGOR ET AL: "Neisseria meningitidis tonB, exbB, and exbD genes: Ton-dependent utilization of protein-bound iron in Neisseriae." JOURNAL OF BACTERIOLOGY, vol. 179, no. 3, 1997, pages 805-812, XP002182142 ISSN: 0021-9193 -& DATABASE EMBL [Online] 17 December 1996 (1996-12-17) retrieved from EBI Database accession no. U77738 XP002182583
- JENNINGS MICHAEL P ET AL: "Cloning and molecular analysis of the Isi1 (rfaF) gene of Neisseria meningitidis which encodes a heptosyl-2-transferase involved in LPS biosynthesis: Evaluation of surface exposed carbohydrates in LPS mediated toxicity for human endothelial cells." MICROBIAL PATHOGENESIS, vol. 19, no. 6, 1995, pages 391-407, XP001022252 ISSN: 0882-4010 -& DATABASE TREMBL [Online] 1 November 1996 (1996-11-01) retrieved from EBI Database accession no. Q51110 XP002182584
- SUN YAO-HUI ET AL: "Functional genomics of Neisseria meningitidis pathogenesis." NATURE MEDICINE, vol. 6, no. 11, November 2000 (2000-11), pages 1269-1273, XP001008318 ISSN: 1078-8956
- DE KLEIJN ESTER D ET AL: "Immunogenicity and safety of a hexavalent meningococcal outer-membrane-vesicle vaccine in children of 2-3 and 7-8 years of age." VACCINE, vol. 18, no. 15, 14 February 2000 (2000-02-14), pages 1456-1466, XP001007679 ISSN: 0264-410X
- VAN DER LEY P ET AL: "Construction of Neisseria meningitidis strains carrying multiple chromosomal copies of the porA gene for use in the production of a multivalent outer membrane vesicle vaccine" VACCINE,BUTTERWORTH SCIENTIFIC. GUILDFORD,GB, vol. 13, no. 4, 1995, pages 401-407, XP004057740 ISSN: 0264-410X
- DIAZ ROMERO J ET AL: "CURRENT STATUS OF MENINGOCOCCAL GROUP B VACCINE CANDIDATES: CAPSULAR OR NONCAPSULAR?" CLINICAL MICROBIOLOGY REVIEWS,US,WASHINGTON, DC, vol. 7, no. 4, 1 October 1994 (1994-10-01), pages 559-575, XP000676353 ISSN: 0893-8512
- ALA'ALDEEN D A A ET AL: "The meningococcal transferrin-binding proteins 1 and 2 are both surface exposed and generate bactericidal antibodies capable of killing homologous and heterologous strains" VACCINE,GB,BUTTERWORTH SCIENTIFIC. GUILDFORD, vol. 14, no. 1, 1996, pages 49-53, XP004057371 ISSN: 0264-410X

### SEQUENCE LISTING

<110> Microscience Limited
<120> Virulence Genes, Proteins, and their Use
<130> REP06436EP
<140> EP01925742.7
   <141> 2001-05-08
<160> 20
<170> Patent In version 3.3
<210> 1
   <211> 2118
   <212> DNA
   <213> Neisseria meningitidis
<220>
   <221> CDS
   <222> (1)..(2118)
<400> 1
   atg ttc gac aaa cac gtt aag acc ttc caa tac ggt aat cag acc gtt      48
   Met Phe Asp Lys His Val Lys Thr Phe Gln Tyr Gly Asn Gln Thr Val
   1               5                   10                  15
   act ttg gaa acc ggc gaa att gcc cgc caa gcc gcc gct gcc gtt aaa      96
   Thr Leu Glu Thr Gly Glu Ile Ala Arg Gln Ala Ala Ala Ala Val Lys
               20                  25                  30
   gtc tct atg ggc gac acc gtg gtt ttg gtt gcc gtt acc acc aac aaa     144
   Val Ser Met Gly Asp Thr Val Val Leu Val Ala Val Thr Thr Asn Lys
           35                  40                  45
   gaa gtg aaa gaa ggc caa gac ttc ttc ccc ctg acc gtc gat tat ttg     192
   Glu Val Lys Glu Gly Gln Asp Phe Phe Pro Leu Thr Val Asp Tyr Leu
       50                  55                  60
   gaa cgc act tac gcc gca ggt aaa atc ccc ggc ggt ttc ttc aaa cgc     240
   Glu Arg Thr Tyr Ala Ala Gly Lys Ile Pro Gly Gly Phe Phe Lys Arg
   65                  70                  75                  80
   gaa ggc aaa caa agc gaa aaa gaa atc ctg acc agc cgt ctg atc gac     288
   Glu Gly Lys Gln Ser Glu Lys Glu Ile Leu Thr Ser Arg Leu Ile Asp
                   85                  90                  95
   cgc ccg att cgt ccg ctg ttc ccc gaa ggt ttc tac cac gac atc caa     336
   Arg Pro Ile Arg Pro Leu Phe Pro Glu Gly Phe Tyr His Asp Ile Gln
               100                 105                 110
   atc gta gcg atg gtc gtg tcc gtc gat ccc gaa atc gat tcc gac att     384
   Ile Val Ala Met Val Val Ser Val Asp Pro Glu Ile Asp Ser Asp Ile
           115                 120                 125
   cct gca atg ttg ggc gcg tct gcc gcg ctg gtg ttg agc ggc gta ccg     432
   Pro Ala Met Leu Gly Ala Ser Ala Ala Leu Val Leu Ser Gly Val Pro
       130                 135                 140
   ttt gcc ggt ccg att ggt gcg gca cgc gtg ggt tat ata aac ggc gtg      480
   Phe Ala Gly Pro Ile Gly Ala Ala Arg Val Gly Tyr Ile Asn Gly Val
   145                 150                 155                 160
   tac gtt ttg aac ccg act aaa gcc gaa ttg gcg aaa tcg caa ttg gac      528
   Tyr Val Leu Asn Pro Thr Lys Ala Glu Leu Ala Lys Ser Gln Leu Asp
                   165                 170                 175
   ttg gtg gtc gcc ggt act tca aaa gcc gtg ttg atg gtg gaa tcc gaa      576
   Leu Val Val Ala Gly Thr Ser Lys Ala Val Leu Met Val Glu Ser Glu
               180                 185                 190
   gcc aaa atc ctg ccc gaa gac gtg atg ctg ggc gcg gtg gtt tac ggc      624
   Ala Lys Ile Leu Pro Glu Asp Val Met Leu Gly Ala Val Val Tyr Gly
           195                 200                 205
   cac gat caa atg cag gtt gcc atc aat gca atc aat gaa ttt gcc gac      672
   His Asp Gln Met Gln Val Ala Ile Asn Ala Ile Asn Glu Phe Ala Asp
       210                 215                 220
   gaa gtc aat ccg gaa ctt tgg gat tgg aaa gca cct gaa acc aat gag      720
   Glu Val Asn Pro Glu Leu Trp Asp Trp Lys Ala Pro Glu Thr Asn Glu
   225                 230                 235                 240
   gaa ctg gtt gcc aaa gtc cgc ggg att gcc ggc gaa acc att aaa gaa      768
   Glu Leu Val Ala Lys Val Arg Gly Ile Ala Gly Glu Thr Ile Lys Glu
                   245                 250                 255
   gcg ttc aaa atc cgt caa aaa caa gcg cgt tct gcc aaa ttg gac gaa      816
   Ala Phe Lys Ile Arg Gln Lys Gln Ala Arg Ser Ala Lys Leu Asp Glu
               260                 265                 270
   gct tgg agt gcg gta aaa gaa gcc ttg att acc gaa gaa acc gac acg      864
   Ala Trp Ser Ala Val Lys Glu Ala Leu Ile Thr Glu Glu Thr Asp Thr
           275                 280                 285
   ctg gca gcc aac gaa atc aaa ggc att ttc aaa cac ttg gaa gcc gat      912
   Leu Ala Ala Asn Glu Ile Lys Gly Ile Phe Lys His Leu Glu Ala Asp
       290                 295                 300
   gtc gtc cgc agc caa att ttg gac ggc caa ccg cgc atc gac ggc cgc      960
   Val Val Arg Ser Gln Ile Leu Asp Gly Gln Pro Arg Ile Asp Gly Arg
   305                 310                 315                 320
   gac acc cgc acc gtc cgt ccg ctg aac atc caa acc ggc gta ttg ccg     1008
   Asp Thr Arg Thr Val Arg Pro Leu Asn Ile Gln Thr Gly Val Leu Pro
                   325                 330                 335
   cgc acg cac ggt tcc gca ttg ttt acc cgt ggc gaa acc caa gcc ctt     1056
   Arg Thr His Gly Ser Ala Leu Phe Thr Arg Gly Glu Thr Gln Ala Leu
               340                 345                 350
   gcc gta gcg act ttg ggt act tcg cgc gac gag caa atc atc gac gcg     1104
   Ala Val Ala Thr Leu Gly Thr Ser Arg Asp Glu Gln Ile Ile Asp Ala
           355                 360                 365
   ctg tcc ggc gaa tac acc gac cgc ttt atg ctg cac tac aac ttt ccg     1152
   Leu Ser Gly Glu Tyr Thr Asp Arg Phe Met Leu His Tyr Asn Phe Pro
       370                 375                 380
   ccg tac tct acc ggc gaa gtg ggc cgc atg ggc gcg ccg aaa cgc cgt      1200
   Pro Tyr Ser Thr Gly Glu Val Gly Arg Met Gly Ala Pro Lys Arg Arg
   385                 390                 395                 400
   gaa atc ggt cac ggt cgt ttg gct aaa cgt gca ttg ttg gcc gta ttg      1248
   Glu Ile Gly His Gly Arg Leu Ala Lys Arg Ala Leu Leu Ala Val Leu
                   405                 410                 415
   ccg aaa cct gaa gat ttc agc tac acc atg cgc gtg gtc tcc gaa att      1296
   Pro Lys Pro Glu Asp Phe Ser Tyr Thr Met Arg Val Val Ser Glu Ile
               420                 425                 430
   acc gaa tcc aac ggc tct tcc tct atg gct tcc gtc tgc ggc ggc tgc      1344
   Thr Glu Ser Asn Gly Ser Ser Ser Met Ala Ser Val Cys Gly Gly Cys
           435                 440                 445
   ttg agc ctg ctg tcc gcc ggc gtg cct ttg aaa gca cac gtt gcc ggt      1392
   Leu Ser Leu Leu Ser Ala Gly Val Pro Leu Lys Ala His Val Ala Gly
       450                 455                 460
   atc gcg atg ggt ctg att ctg gaa ggc aat aag ttt gcc gtc ctg acc      1440
   Ile Ala Met Gly Leu Ile Leu Glu Gly Asn Lys Phe Ala Val Leu Thr
   465                 470                 475                 480
   gac att ttg ggc gac gaa gac cac ttg ggc gat atg gac ttt aaa gtg      1488
   Asp Ile Leu Gly Asp Glu Asp His Leu Gly Asp Met Asp Phe Lys Val
                   485                 490                 495
   gct ggt acg acc gaa ggc gtt acc gcg ctg caa atg gac atc aaa atc      1536
   Ala Gly Thr Thr Glu Gly Val Thr Ala Leu Gln Met Asp Ile Lys Ile
               500                 505                 510
   caa ggc att acc aaa gag att atg caa atc gct ttg gcg cag gcc aaa      1584
   Gln Gly Ile Thr Lys Glu Ile Met Gln Ile Ala Leu Ala Gln Ala Lys
           515                 520                 525
   gaa gcg cgt ctg cac atc ttg gat cag atg aaa gcc gcc gtt gcg ggc      1632
   Glu Ala Arg Leu His Ile Leu Asp Gln Met Lys Ala Ala Val Ala Gly
       530                 535                 540
   ccg caa gag ctg tcc gca cac gcg cca cgc ttg ttc acg atg aaa atc      1680
   Pro Gln Glu Leu Ser Ala His Ala Pro Arg Leu Phe Thr Met Lys Ile
   545                 550                 555                 560
   aac caa gac aaa atc cgc gaa gtt atc ggt aag ggc ggt gaa acc atc      1728
   Asn Gln Asp Lys Ile Arg Glu Val Ile Gly Lys Gly Gly Glu Thr Ile
                   565                 570                 575
   cgt gcg att acc gct gaa acc ggt acg gaa atc aat att gcc gaa gac      1776
   Arg Ala Ile Thr Ala Glu Thr Gly Thr Glu Ile Asn Ile Ala Glu Asp
               580                 585                 590
   ggt acg att acc att gcc gca acc act caa gaa gcc ggc gat gcg gcg      1824
   Gly Thr Ile Thr Ile Ala Ala Thr Thr Gln Glu Ala Gly Asp Ala Ala
           595                 600                 605
   aaa aaa cgc atc gag cag att act gcc gaa gtg gaa gtg ggc aaa gtg      1872
   Lys Lys Arg Ile Glu Gln Ile Thr Ala Glu Val Glu Val Gly Lys Val
       610                 615                 620
   tac gaa ggc act gtg gtg aaa atc ctc gac aac aat gtc ggc gcg att      1920
   Tyr Glu Gly Thr Val Val Lys Ile Leu Asp Asn Asn Val Gly Ala Ile
   625                 630                 635                 640
   gtc agt gtg atg ccg ggc aaa gac ggt ttg gta cac atc agc caa atc      1968
   Val Ser Val Met Pro Gly Lys Asp Gly Leu Val His Ile Ser Gln Ile
                   645                 650                 655
   gcc cac gag cgc gta cgc aat gtc ggc gac tac ctg caa gtc ggt cag      2016
   Ala His Glu Arg Val Arg Asn Val Gly Asp Tyr Leu Gln Val Gly Gln
               660                 665                 670
   gtg gtg aac gtg aaa gca ttg gaa gtg gac gac aga ggc cgt gtc cgt      2064
   Val Val Asn Val Lys Ala Leu Glu Val Asp Asp Arg Gly Arg Val Arg
           675                 680                 685
   ctg tcc atc aaa gcc ctg ctg gac gcg cct gcc cgt gag gaa aat gcc      2112
   Leu Ser Ile Lys Ala Leu Leu Asp Ala Pro Ala Arg Glu Glu Asn Ala
       690                 695                 700
   gcc gag                                                              2118
   Ala Glu
   705
<210> 2
   <211> 706
   <212> PRT
   <213> Neisseria meningitidis
<400> 2
   Met Phe Asp Lys His Val Lys Thr Phe Gln Tyr Gly Asn Gln Thr Val
   1               5                   10                  15
   Thr Leu Glu Thr Gly Glu Ile Ala Arg Gln Ala Ala Ala Ala Val Lys
               20                  25                  30
   Val Ser Met Gly Asp Thr Val Val Leu Val Ala Val Thr Thr Asn Lys
           35                  40                  45
   Glu Val Lys Glu Gly Gln Asp Phe Phe Pro Leu Thr Val Asp Tyr Leu
       50                  55                  60
   Glu Arg Thr Tyr Ala Ala Gly Lys Ile Pro Gly Gly Phe Phe Lys Arg
   65                  70                  75                  80
   Glu Gly Lys Gln Ser Glu Lys Glu Ile Leu Thr Ser Arg Leu Ile Asp
                   85                  90                  95
   Arg Pro Ile Arg Pro Leu Phe Pro Glu Gly Phe Tyr His Asp Ile Gln
               100                 105                 110
   Ile Val Ala Met Val Val Ser Val Asp Pro Glu Ile Asp Ser Asp Ile
           115                 120                 125
   Pro Ala Met Leu Gly Ala Ser Ala Ala Leu Val Leu Ser Gly Val Pro
       130                 135                 140
   Phe Ala Gly Pro Ile Gly Ala Ala Arg Val Gly Tyr Ile Asn Gly Val
   145                 150                 155                 160
   Tyr Val Leu Asn Pro Thr Lys Ala Glu Leu Ala Lys Ser Gln Leu Asp
                   165                 170                 175
   Leu Val Val Ala Gly Thr Ser Lys Ala Val Leu Met Val Glu Ser Glu
               180                 185                 190
   Ala Lys Ile Leu Pro Glu Asp Val Met Leu Gly Ala Val Val Tyr Gly
           195                 200                 205
   His Asp Gln Met Gln Val Ala Ile Asn Ala Ile Asn Glu Phe Ala Asp
       210                 215                 220
   Glu Val Asn Pro Glu Leu Trp Asp Trp Lys Ala Pro Glu Thr Asn Glu
   225                 230                 235                 240
   Glu Leu Val Ala Lys Val Arg Gly Ile Ala Gly Glu Thr Ile Lys Glu
                   245                 250                 255
   Ala Phe Lys Ile Arg Gln Lys Gln Ala Arg Ser Ala Lys Leu Asp Glu
               260                 265                 270
   Ala Trp Ser Ala Val Lys Glu Ala Leu Ile Thr Glu Glu Thr Asp Thr
           275                 280                 285
   Leu Ala Ala Asn Glu Ile Lys Gly Ile Phe Lys His Leu Glu Ala Asp
       290                 295                 300
   Val Val Arg Ser Gln Ile Leu Asp Gly Gln Pro Arg Ile Asp Gly Arg
   305                 310                 315                 320
   Asp Thr Arg Thr Val Arg Pro Leu Asn Ile Gln Thr Gly Val Leu Pro
                   325                 330                 335
   Arg Thr His Gly Ser Ala Leu Phe Thr Arg Gly Glu Thr Gln Ala Leu
               340                 345                 350
   Ala Val Ala Thr Leu Gly Thr Ser Arg Asp Glu Gln Ile Ile Asp Ala
           355                 360                 365
   Leu Ser Gly Glu Tyr Thr Asp Arg Phe Met Leu His Tyr Asn Phe Pro
       370                 375                 380
   Pro Tyr Ser Thr Gly Glu Val Gly Arg Met Gly Ala Pro Lys Arg Arg
   385                 390                 395                 400
   Glu Ile Gly His Gly Arg Leu Ala Lys Arg Ala Leu Leu Ala Val Leu
                   405                 410                 415
   Pro Lys Pro Glu Asp Phe Ser Tyr Thr Met Arg Val Val Ser Glu Ile
               420                 425                 430
   Thr Glu Ser Asn Gly Ser Ser Ser Met Ala Ser Val Cys Gly Gly Cys
           435                 440                 445
   Leu Ser Leu Leu Ser Ala Gly Val Pro Leu Lys Ala His Val Ala Gly
       450                 455                 460
   Ile Ala Met Gly Leu Ile Leu Glu Gly Asn Lys Phe Ala Val Leu Thr
   465                 470                 475                 480
   Asp Ile Leu Gly Asp Glu Asp His Leu Gly Asp Met Asp Phe Lys Val
                   485                 490                 495
   Ala Gly Thr Thr Glu Gly Val Thr Ala Leu Gln Met Asp Ile Lys Ile
               500                 505                 510
   Gln Gly Ile Thr Lys Glu Ile Met Gln Ile Ala Leu Ala Gln Ala Lys
           515                 520                 525
   Glu Ala Arg Leu His Ile Leu Asp Gln Met Lys Ala Ala Val Ala Gly
       530                 535                 540
   Pro Gln Glu Leu Ser Ala His Ala Pro Arg Leu Phe Thr Met Lys Ile
   545                 550                 555                 560
   Asn Gln Asp Lys Ile Arg Glu Val Ile Gly Lys Gly Gly Glu Thr Ile
                   565                 570                 575
   Arg Ala Ile Thr Ala Glu Thr Gly Thr Glu Ile Asn Ile Ala Glu Asp
               580                 585                 590
   Gly Thr Ile Thr Ile Ala Ala Thr Thr Gln Glu Ala Gly Asp Ala Ala
           595                 600                 605
   Lys Lys Arg Ile Glu Gln Ile Thr Ala Glu Val Glu Val Gly Lys Val
       610                 615                 620
   Tyr Glu Gly Thr Val Val Lys Ile Leu Asp Asn Asn Val Gly Ala Ile
   625                 630                 635                 640
   Val Ser Val Met Pro Gly Lys Asp Gly Leu Val His Ile Ser Gln Ile
                   645                 650                 655
   Ala His Glu Arg Val Arg Asn Val Gly Asp Tyr Leu Gln Val Gly Gln
               660                 665                 670
   Val Val Asn Val Lys Ala Leu Glu Val Asp Asp Arg Gly Arg Val Arg
           675                 680                 685
   Leu Ser Ile Lys Ala Leu Leu Asp Ala Pro Ala Arg Glu Glu Asn Ala
       690                 695                 700
   Ala Glu
   705
<210> 3
   <211> 1686
   <212> DNA
   <213> Neisseria meningitidis
<220>
   <221> CDS
   <222> (1)..(1686)
<400> 3
   atg tta caa tcc gaa aat tcg aaa aat tta atc tct tgt tca ata aag        48
   Met Leu Gln Ser Glu Asn Ser Lys Asn Leu Ile Ser Cys Ser Ile Lys
   1               5                   10                  15
   gct tta cca atc atg att tct acc aac ggc atc acc atg cag ttc ggc        96
   Ala Leu Pro Ile Met Ile Ser Thr Asn Gly Ile Thr Met Gln Phe Gly
               20                  25                  30
   gcg aag ccg ctg ttt gaa aac gta tcc gtt aaa ttc ggc gaa ggc aac       144
   Ala Lys Pro Leu Phe Glu Asn Val Ser Val Lys Phe Gly Glu Gly Asn
           35                  40                  45
   cgc tac ggt ttg atc ggc gcg aac ggc tca ggc aaa tcc acc ttc atg       192
   Arg Tyr Gly Leu Ile Gly Ala Asn Gly Ser Gly Lys Ser Thr Phe Met
       50                  55                  60
   aaa atc ctc ggc ggc gat ttg gaa cag aca gcc ggc gaa gtg gcg att       240
   Lys Ile Leu Gly Gly Asp Leu Glu Gln Thr Ala Gly Glu Val Ala Ile
   65                  70                  75                  80
   gaa aac ggc gtg cgt ttg ggt aaa ttg cgc caa gac cag ttt gcc tac       288
   Glu Asn Gly Val Arg Leu Gly Lys Leu Arg Gln Asp Gln Phe Ala Tyr
                   85                  90                  95
   gaa gat atg cgc gtg ctg gac gtg gta atg atg ggt cac acc gaa atg     336
   Glu Asp Met Arg Val Leu Asp Val Val Met Met Gly His Thr Glu Met
               100                 105                 110
   tgg gcg gcg atg acc gaa cgt gat gcg att tac gcc aat ccc gaa gcc     384
   Trp Ala Ala Met Thr Glu Arg Asp Ala Ile Tyr Ala Asn Pro Glu Ala
           115                 120                 125
   acc gaa gac gac tac atg aaa gcc gca gaa ctg gaa gcc aag ttc gcc     432
   Thr Glu Asp Asp Tyr Met Lys Ala Ala Glu Leu Glu Ala Lys Phe Ala
       130                 135                 140
   gaa tac gac ggc tac acc gcc gaa gcg cgt gcc gcc gag ttg ttg agc     480
   Glu Tyr Asp Gly Tyr Thr Ala Glu Ala Arg Ala Ala Glu Leu Leu Ser
   145                 150                 155                 160
   ggc gtg ggc att tcc gaa gat ttg cac aat gca acc atg gca gaa gtt     528
   Gly Val Gly Ile Ser Glu Asp Leu His Asn Ala Thr Met Ala Glu Val
                   165                 170                 175
   gcc ccg ggc ttc aaa ctg cgc gta ttg ctg gcg caa gcc ctg ttc tcc     576
   Ala Pro Gly Phe Lys Leu Arg Val Leu Leu Ala Gln Ala Leu Phe Ser
               180                 185                 190
   aag ccg gat gta ttg ctc ttg gac gaa ccg acc aat aac ttg gac att     624
   Lys Pro Asp Val Leu Leu Leu Asp Glu Pro Thr Asn Asn Leu Asp Ile
           195                 200                 205
   aat acc atc cgt tgg ttg gaa ggc gtg ttg aac cag tac gac tcc acg     672
   Asn Thr Ile Arg Trp Leu Glu Gly Val Leu Asn Gln Tyr Asp Ser Thr
       210                 215                 220
   atg att atc atc tcg cac gac cgc cac ttc ttg aac gaa gtc tgc acg     720
   Met Ile Ile Ile Ser His Asp Arg His Phe Leu Asn Glu Val Cys Thr
   225                 230                 235                 240
   cat atg gca gat ttg gat tac aac acc atc acc atc tat ccg ggc aac     768
   His Met Ala Asp Leu Asp Tyr Asn Thr Ile Thr Ile Tyr Pro Gly Asn
                   245                 250                 255
   tac gac gac tat atg ctc gcc tcc gcc caa tcg cgc gaa cgc gcc ctg     816
   Tyr Asp Asp Tyr Met Leu Ala Ser Ala Gln Ser Arg Glu Arg Ala Leu
               260                 265                 270
   aaa gac aat gcc aag gcg aag gaa aaa ttg cag gaa ttg caa gaa ttt     864
   Lys Asp Asn Ala Lys Ala Lys Glu Lys Leu Gln Glu Leu Gln Glu Phe
           275                 280                 285
   gtg gcg cgg ttc agt gcc aac aaa tcc aaa gcc cgt cag gca acc agc     912
   Val Ala Arg Phe Ser Ala Asn Lys Ser Lys Ala Arg Gln Ala Thr Ser
       290                 295                 300
   cgt ctg aaa cag gca gac aaa atc aaa tcg gag atg gtc gag gtc aaa     960
   Arg Leu Lys Gln Ala Asp Lys Ile Lys Ser Glu Met Val Glu Val Lys
   305                 310                 315                 320
   ccc tct acc cgt caa aac ccg tat atc cgt ttt gaa gcc gat gaa aaa    1008
   Pro Ser Thr Arg Gln Asn Pro Tyr Ile Arg Phe Glu Ala Asp Glu Lys
                   325                 330                 335
   gcc aag ctg cac cgt cag gct gtg gaa gtt gaa aaa ctg gcg aaa cgc      1056
   Ala Lys Leu His Arg Gln Ala Val Glu Val Glu Lys Leu Ala Lys Arg
               340                 345                 350
   ttt gaa acc cag ttg ttt aaa aac ctg aac ttt atc ctt gaa gcg gga      1104
   Phe Glu Thr Gln Leu Phe Lys Asn Leu Asn Phe Ile Leu Glu Ala Gly
           355                 360                 365
   caa cgc ctc gcc atc atc ggc ccg aac ggc gcg ggc aaa tcc acc ctg      1152
   Gln Arg Leu Ala Ile Ile Gly Pro Asn Gly Ala Gly Lys Ser Thr Leu
       370                 375                 380
   ctg aaa ctc ttg gcc ggc gcg tac aac ccc gaa tat tca gac ggt ctg      1200
   Leu Lys Leu Leu Ala Gly Ala Tyr Asn Pro Glu Tyr Ser Asp Gly Leu
   385                 390                 395                 400
   ctg ccg gac gaa ggc agc atc aaa tgg gcg gaa aaa gcc agc gtc ggc      1248
   Leu Pro Asp Glu Gly Ser Ile Lys Trp Ala Glu Lys Ala Ser Val Gly
                   405                 410                 415
   tac tat ccg caa gat cac gaa aac gac ttc gat gtc gat atg gat ttg      1296
   Tyr Tyr Pro Gln Asp His Glu Asn Asp Phe Asp Val Asp Met Asp Leu
               420                 425                 430
   agc gaa tgg atg cgc caa tgg ggg cag gac ggc gac gac gaa caa gtc      1344
   Ser Glu Trp Met Arg Gln Trp Gly Gln Asp Gly Asp Asp Glu Gln Val
           435                 440                 445
   atc cgc ggc act ttg ggg cgt ttg ctc ttc ggc agt aac gat gtc gtg      1392
   Ile Arg Gly Thr Leu Gly Arg Leu Leu Phe Gly Ser Asn Asp Val Val
       450                 455                 460
   aaa aaa gtg aag gtt ctc tcc ggt ggt gaa aaa ggc cgt atg ctt tac      1440
   Lys Lys Val Lys Val Leu Ser Gly Gly Glu Lys Gly Arg Met Leu Tyr
   465                 470                 475                 480
   ggc aaa ctg ttg ctg ttg aaa ccc aat gtc tta gtc atg gat gaa ccg      1488
   Gly Lys Leu Leu Leu Leu Lys Pro Asn Val Leu Val Met Asp Glu Pro
                   485                 490                 495
   acc aac cac atg gac atg gaa agc atc gaa tcc ttg aac atg gcg ctg      1536
   Thr Asn His Met Asp Met Glu Ser Ile Glu Ser Leu Asn Met Ala Leu
               500                 505                 510
   gaa aaa tac aac ggc acg ctg att ttc gtc tcc cac gac cgt cag ttc      1584
   Glu Lys Tyr Asn Gly Thr Leu Ile Phe Val Ser His Asp Arg Gln Phe
           515                 520                 525
   gtt tct tcc ctg gct acc caa atc att gaa ttg gac ggc aaa ggc gga      1632
   Val Ser Ser Leu Ala Thr Gln Ile Ile Glu Leu Asp Gly Lys Gly Gly
       530                 535                 540
   tat gaa cac tac ttg ggc gat tac gaa agc tat ctc gag aaa aaa ggc      1680
   Tyr Glu His Tyr Leu Gly Asp Tyr Glu Ser Tyr Leu Glu Lys Lys Gly
   545                 550                 555                 560
   gta gca                                                              1686
   Val Ala
<210> 4
   <211> 562
   <212> PRT
   <213> Neisseria meningitidis
<400> 4
   Met Leu Gln Ser Glu Asn Ser Lys Asn Leu Ile Ser Cys Ser Ile Lys
   1 5 10 15
   Ala Leu Pro Ile Met Ile Ser Thr Asn Gly Ile Thr Met Gln Phe Gly
   20 25 30
   Ala Lys Pro Leu Phe Glu Asn Val Ser Val Lys Phe Gly Glu Gly Asn
   35 40 45
   Arg Tyr Gly Leu Ile Gly Ala Asn Gly Ser Gly Lys Ser Thr Phe Met
   50 55 60
   Lys Ile Leu Gly Gly Asp Leu Glu Gln Thr Ala Gly Glu Val Ala Ile
   65 70 75 80
   Glu Asn Gly Val Arg Leu Gly Lys Leu Arg Gln Asp Gln Phe Ala Tyr
   85 90 95
   Glu Asp Met Arg Val Leu Asp Val Val Met Met Gly His Thr Glu Met
   100 105 110
   Trp Ala Ala Met Thr Glu Arg Asp Ala Ile Tyr Ala Asn Pro Glu Ala
   115 120 125
   Thr Glu Asp Asp Tyr Met Lys Ala Ala Glu Leu Glu Ala Lys Phe Ala
   130 135 140
   Glu Tyr Asp Gly Tyr Thr Ala Glu Ala Arg Ala Ala Glu Leu Leu Ser
   145 150 155 160
   Gly Val Gly Ile Ser Glu Asp Leu His Asn Ala Thr Met Ala Glu Val
   165 170 175
   Ala Pro Gly Phe Lys Leu Arg Val Leu Leu Ala Gln Ala Leu Phe Ser
   180 185 190
   Lys Pro Asp Val Leu Leu Leu Asp Glu Pro Thr Asn Asn Leu Asp Ile
   195 200 205
   Asn Thr Ile Arg Trp Leu Glu Gly Val Leu Asn Gln Tyr Asp Ser Thr
       210                 215                 220
   Met Ile Ile Ile Ser His Asp Arg His Phe Leu Asn Glu Val Cys Thr
   225                 230                 235                 240
   His Met Ala Asp Leu Asp Tyr Asn Thr Ile Thr Ile Tyr Pro Gly Asn
                   245                 250                 255
   Tyr Asp Asp Tyr Met Leu Ala Ser Ala Gln Ser Arg Glu Arg Ala Leu
               260                 265                 270
   Lys Asp Asn Ala Lys Ala Lys Glu Lys Leu Gln Glu Leu Gln Glu Phe
           275                 280                 285
   Val Ala Arg Phe Ser Ala Asn Lys Ser Lys Ala Arg Gln Ala Thr Ser
       290                 295                 300
   Arg Leu Lys Gln Ala Asp Lys Ile Lys Ser Glu Met Val Glu Val Lys
   305                 310                 315                 320
   Pro Ser Thr Arg Gln Asn Pro Tyr Ile Arg Phe Glu Ala Asp Glu Lys
                   325                 330                 335
   Ala Lys Leu His Arg Gln Ala Val Glu Val Glu Lys Leu Ala Lys Arg
               340                 345                 350
   Phe Glu Thr Gln Leu Phe Lys Asn Leu Asn Phe Ile Leu Glu Ala Gly
           355                 360                 365
   Gln Arg Leu Ala Ile Ile Gly Pro Asn Gly Ala Gly Lys Ser Thr Leu
       370                 375                 380
   Leu Lys Leu Leu Ala Gly Ala Tyr Asn Pro Glu Tyr Ser Asp Gly Leu
   385                 390                 395                 400
   Leu Pro Asp Glu Gly Ser Ile Lys Trp Ala Glu Lys Ala Ser Val Gly
                   405                 410                 415
   Tyr Tyr Pro Gln Asp His Glu Asn Asp Phe Asp Val Asp Met Asp Leu
               420                 425                 430
   Ser Glu Trp Met Arg Gln Trp Gly Gln Asp Gly Asp Asp Glu Gln Val
           435                 440                 445
   Ile Arg Gly Thr Leu Gly Arg Leu Leu Phe Gly Ser Asn Asp Val Val
       450                 455                 460
   Lys Lys Val Lys Val Leu Ser Gly Gly Glu Lys Gly Arg Met Leu Tyr
   465                 470                 475                 480
   Gly Lys Leu Leu Leu Leu Lys Pro Asn Val Leu Val Met Asp Glu Pro
                   485                 490                 495
   Thr Asn His Met Asp Met Glu Ser Ile Glu Ser Leu Asn Met Ala Leu
               500                 505                 510
   Glu Lys Tyr Asn Gly Thr Leu Ile Phe Val Ser His Asp Arg Gln Phe
           515                 520                 525
   Val Ser Ser Leu Ala Thr Gln Ile Ile Glu Leu Asp Gly Lys Gly Gly
       530                 535                 540
   Tyr Glu His Tyr Leu Gly Asp Tyr Glu Ser Tyr Leu Glu Lys Lys Gly
   545                 550                 555                 560
   Val Ala
<210> 5
   <211> 1476
   <212> DNA
   <213> Neisseria meningitidis
<220>
   <221> CDS
   <222> (1)..(1476)
<400> 5
   atg aaa tac aaa gac ctg cgc gac ttc atc gcc atg ctc gag cag cag       48
   Met Lys Tyr Lys Asp Leu Arg Asp Phe Ile Ala Met Leu Glu Gln Gln
   1               5                   10                  15
   ggc aaa ctc aaa cgc atc gcg cac ccc gtt tcc ccg cat ttg gaa atg       96
   Gly Lys Leu Lys Arg Ile Ala His Pro Val Ser Pro His Leu Glu Met
               20                  25                  30
   acc gaa atc gcc gac cgc gtg ctg cgc gcc gaa ggg ccg gcg ttg ttg      144
   Thr Glu Ile Ala Asp Arg Val Leu Arg Ala Glu Gly Pro Ala Leu Leu
           35                  40                  45
   ttt gaa cac cca gtt aag ccc gac ggt acg cgc tat gat tat ccc gtg      192
   Phe Glu His Pro Val Lys Pro Asp Gly Thr Arg Tyr Asp Tyr Pro Val
       50                  55                  60
   ttg gca aac ctg ttc ggc acg ccc gaa cgt gtg gcg atg ggc atg ggc      240
   Leu Ala Asn Leu Phe Gly Thr Pro Glu Arg Val Ala Met Gly Met Gly
   65                  70                  75                  80
   gcg gac agc gtg tcc aag ctg cgc gaa atc ggg cag acg ctg gcg tat      288
   Ala Asp Ser Val Ser Lys Leu Arg Glu Ile Gly Gln Thr Leu Ala Tyr
                   85                  90                  95
   ttg aaa gaa ccc gaa ccg ccc aaa ggc att aaa gac gcg ttt tcc aaa      336
   Leu Lys Glu Pro Glu Pro Pro Lys Gly Ile Lys Asp Ala Phe Ser Lys
               100                 105                 110
   ctg ccg ctc ttg aaa gac att tgg agc atg gcg ccg aac gtg gtg aaa      384
   Leu Pro Leu Leu Lys Asp Ile Trp Ser Met Ala Pro Asn Val Val Lys
           115                 120                 125
   aat gcg ccg tgt cag gaa atc gta tgg gaa ggc gaa gac gtt gat ttg      432
   Asn Ala Pro Cys Gln Glu Ile Val Trp Glu Gly Glu Asp Val Asp Leu
       130                 135                 140
   tat caa ctt ccg att cag cat tgc tgg ccg gaa gac gtt gcg ccg ctg      480
   Tyr Gln Leu Pro Ile Gln His Cys Trp Pro Glu Asp Val Ala Pro Leu
   145                 150                 155                 160
   gta acg tgg ggc ttg acc gtc acg cgc ggg ccg cac aaa aaa cgc caa      528
   Val Thr Trp Gly Leu Thr Val Thr Arg Gly Pro His Lys Lys Arg Gln
                   165                 170                 175
   aat ctc ggc att tac cgc caa caa tta atc ggc ata aac aag ctg att      576
   Asn Leu Gly Ile Tyr Arg Gln Gln Leu Ile Gly Ile Asn Lys Leu Ile
               180                 185                 190
   atg cgc tgg ctg tcg cat cgc ggc ggc gcg ctg gat tat cag gaa ttc      624
   Met Arg Trp Leu Ser His Arg Gly Gly Ala Leu Asp Tyr Gln Glu Phe
           195                 200                 205
   cgc aaa ctc aat ccc gat acg ccg tat ccc gtc gcc gtc gtg ctc ggc      672
   Arg Lys Leu Asn Pro Asp Thr Pro Tyr Pro Val Ala Val Val Leu Gly
       210                 215                 220
   tgc gac cct gcc acc att ttg ggc gcg gta acg ccc gtt ccc gat acc      720
   Cys Asp Pro Ala Thr Ile Leu Gly Ala Val Thr Pro Val Pro Asp Thr
   225                 230                 235                 240
   ttg agc gaa tac cag ttt gcc gga ctg ctg cgc ggt tcg cgg acg gaa      768
   Leu Ser Glu Tyr Gln Phe Ala Gly Leu Leu Arg Gly Ser Arg Thr Glu
                   245                 250                 255
   ctg gtg aaa tgt atc ggc aac gat ttg caa gtg cct gcc cgc gca gaa      816
   Leu Val Lys Cys Ile Gly Asn Asp Leu Gln Val Pro Ala Arg Ala Glu
               260                 265                 270
   atc gtg ttg gaa ggc gtc atc cat cca aac gaa acc gcg ttg gaa ggt      864
   Ile Val Leu Glu Gly Val Ile His Pro Asn Glu Thr Ala Leu Glu Gly
           275                 280                 285
   cca tac ggc gac cac acg ggc tat tac aac gag cag gac cat ttc ccc      912
   Pro Tyr Gly Asp His Thr Gly Tyr Tyr Asn Glu Gln Asp His Phe Pro
       290                 295                 300
   gtg ttt acg gtc gaa cgc atc acc atg cgc gaa aac ccg att tac cac      960
   Val Phe Thr Val Glu Arg Ile Thr Met Arg Glu Asn Pro Ile Tyr His
   305                 310                 315                 320
   tct acc tac aca ggc aaa ccg ccc gat gaa ccc gcc gtt ttg ggc gtg     1008
   Ser Thr Tyr Thr Gly Lys Pro Pro Asp Glu Pro Ala Val Leu Gly Val
                   325                 330                 335
   gcg ttg aac gaa gtg ttc gta ccg ctt ttg caa aag cag ttc ccc gaa      1056
   Ala Leu Asn Glu Val Phe Val Pro Leu Leu Gln Lys Gln Phe Pro Glu
               340                 345                 350
   atc acc gat ttt tac ctg ccg ccc gaa ggc tgc tcc tac cgc atg gcg      1104
   Ile Thr Asp Phe Tyr Leu Pro Pro Glu Gly Cys Ser Tyr Arg Met Ala
           355                 360                 365
   gtg gtg agc atg aaa aaa cag tac gcc gga cac gcc aag cgc gtg atg      1152
   Val Val Ser Met Lys Lys Gln Tyr Ala Gly His Ala Lys Arg Val Met
       370                 375                 380
   atg ggc tgc tgg tcg ttc ctg cgc cag ttt atg tac acc aaa ttc atc      1200
   Met Gly Cys Trp Ser Phe Leu Arg Gln Phe Met Tyr Thr Lys Phe Ile
   385                 390                 395                 400
   att gtg gtg gat gac gat gtg gat gtg cgc gac tgg aaa gaa gtc atc      1248
   Ile Val Val Asp Asp Asp Val Asp Val Arg Asp Trp Lys Glu Val Ile
                   405                 410                 415
   tgg gcg gta acc acg cgc atg gac ccc gtg cgc gat acc gtt ttg atg      1296
   Trp Ala Val Thr Thr Arg Met Asp Pro Val Arg Asp Thr Val Leu Met
               420                 425                 430
   gaa aac acg ccc atc gac tac ctc gac ttc gcc agc ccc gtc agc gga      1344
   Glu Asn Thr Pro Ile Asp Tyr Leu Asp Phe Ala Ser Pro Val Ser Gly
           435                 440                 445
   ctt ggc ggc aaa atg ggt ttg gat gcg acc aac aag tgg ccg ggc gaa      1392
   Leu Gly Gly Lys Met Gly Leu Asp Ala Thr Asn Lys Trp Pro Gly Glu
       450                 455                 460
   acc gac cgc gaa tgg gga cgg gtg att aaa aaa gac cct gcg gtt acg      1440
   Thr Asp Arg Glu Trp Gly Arg Val Ile Lys Lys Asp Pro Ala Val Thr
   465                 470                 475                 480
   gct aag att gat gag att tgg gag gaa ttg ggg ttg                      1476
   Ala Lys Ile Asp Glu Ile Trp Glu Glu Leu Gly Leu
                   485                 490
<210> 6
   <211> 492
   <212> PRT
   <213> Neisseria meningitidis
<400> 6
   Met Lys Tyr Lys Asp Leu Arg Asp Phe Ile Ala Met Leu Glu Gln Gln
   1               5                   10                  15
   Gly Lys Leu Lys Arg Ile Ala His Pro Val Ser Pro His Leu Glu Met
               20                  25                  30
   Thr Glu Ile Ala Asp Arg Val Leu Arg Ala Glu Gly Pro Ala Leu Leu
           35                  40                  45
   Phe Glu His Pro Val Lys Pro Asp Gly Thr Arg Tyr Asp Tyr Pro Val
       50                  55                  60
   Leu Ala Asn Leu Phe Gly Thr Pro Glu Arg Val Ala Met Gly Met Gly
   65                  70                  75                  80
   Ala Asp Ser Val Ser Lys Leu Arg Glu Ile Gly Gln Thr Leu Ala Tyr
                   85                  90                  95
   Leu Lys Glu Pro Glu Pro Pro Lys Gly Ile Lys Asp Ala Phe Ser Lys
               100                 105                 110
   Leu Pro Leu Leu Lys Asp Ile Trp Ser Met Ala Pro Asn Val Val Lys
           115                 120                 125
   Asn Ala Pro Cys Gln Glu Ile Val Trp Glu Gly Glu Asp Val Asp Leu
       130                 135                 140
   Tyr Gln Leu Pro Ile Gln His Cys Trp Pro Glu Asp Val Ala Pro Leu
   145                 150                 155                 160
   Val Thr Trp Gly Leu Thr Val Thr Arg Gly Pro His Lys Lys Arg Gln
                   165                 170                 175
   Asn Leu Gly Ile Tyr Arg Gln Gln Leu Ile Gly Ile Asn Lys Leu Ile
               180                 185                 190
   Met Arg Trp Leu Ser His Arg Gly Gly Ala Leu Asp Tyr Gln Glu Phe
           195                 200                 205
   Arg Lys Leu Asn Pro Asp Thr Pro Tyr Pro Val Ala Val Val Leu Gly
       210                 215                 220
   Cys Asp Pro Ala Thr Ile Leu Gly Ala Val Thr Pro Val Pro Asp Thr
   225                 230                 235                 240
   Leu Ser Glu Tyr Gln Phe Ala Gly Leu Leu Arg Gly Ser Arg Thr Glu
                   245                 250                 255
   Leu Val Lys Cys Ile Gly Asn Asp Leu Gln Val Pro Ala Arg Ala Glu
               260                 265                 270
   Ile Val Leu Glu Gly Val Ile His Pro Asn Glu Thr Ala Leu Glu Gly
           275                 280                 285
   Pro Tyr Gly Asp His Thr Gly Tyr Tyr Asn Glu Gln Asp His Phe Pro
       290                 295                 300
   Val Phe Thr Val Glu Arg Ile Thr Met Arg Glu Asn Pro Ile Tyr His
   305                 310                 315                 320
   Ser Thr Tyr Thr Gly Lys Pro Pro Asp Glu Pro Ala Val Leu Gly Val
                   325                 330                 335
   Ala Leu Asn Glu Val Phe Val Pro Leu Leu Gln Lys Gln Phe Pro Glu
               340                 345                 350
   Ile Thr Asp Phe Tyr Leu Pro Pro Glu Gly Cys Ser Tyr Arg Met Ala
           355                 360                 365
   Val Val Ser Met Lys Lys Gln Tyr Ala Gly His Ala Lys Arg Val Met
       370                 375                 380
   Met Gly Cys Trp Ser Phe Leu Arg Gln Phe Met Tyr Thr Lys Phe Ile
   385                 390                 395                 400
   Ile Val Val Asp Asp Asp Val Asp Val Arg Asp Trp Lys Glu Val Ile
                   405                 410                 415
   Trp Ala Val Thr Thr Arg Met Asp Pro Val Arg Asp Thr Val Leu Met
               420                 425                 430
   Glu Asn Thr Pro Ile Asp Tyr Leu Asp Phe Ala Ser Pro Val Ser Gly
           435                 440                 445
   Leu Gly Gly Lys Met Gly Leu Asp Ala Thr Asn Lys Trp Pro Gly Glu
       450                 455                 460
   Thr Asp Arg Glu Trp Gly Arg Val Ile Lys Lys Asp Pro Ala Val Thr
   465                 470                 475                 480
   Ala Lys Ile Asp Glu Ile Trp Glu Glu Leu Gly Leu
                   485                 490
<210> 7
   <211> 1257
   <212> DNA
   <213> Neisseria meningitidis
<220>
   <221> CDS
   <222> (1)..(1257)
<400> 7
   atg aaa cag acc gtc ctc aaa aat aac ctg caa aac ctg ctt gaa agc      48
   Met Lys Gln Thr Val Leu Lys Asn Asn Leu Gln Asn Leu Leu Glu Ser
   1               5                   10                  15
   gca gaa aat atc ctg ctg ctt caa ggc cct gtc ggc gat ttt ttt ctg      96
   Ala Glu Asn Ile Leu Leu Leu Gln Gly Pro Val Gly Asp Phe Phe Leu
               20                  25                  30
   cgc ctt gcc gac tgg ctg act gca aac ggc aaa acc gta cat aaa ttc      144
   Arg Leu Ala Asp Trp Leu Thr Ala Asn Gly Lys Thr Val His Lys Phe
           35                  40                  45
   aac ttt aat gca ggc gac gac tat ttt tat ccg ccc act caa gcg cat      192
   Asn Phe Asn Ala Gly Asp Asp Tyr Phe Tyr Pro Pro Thr Gln Ala His
       50                  55                  60
   acc gtt gtt ttt aac gac aac tac gat gcc ttt cct gag ttt ttg caa      240
   Thr Val Val Phe Asn Asp Asn Tyr Asp Ala Phe Pro Glu Phe Leu Gln
   65                  70                  75                  80
   gaa tac atc act caa cat cac atc cag gcc gtt gtc tgc ttt ggc gac      288
   Glu Tyr Ile Thr Gln His His Ile Gln Ala Val Val Cys Phe Gly Asp
                   85                  90                  95
   aca cgc cct tat cac gtc att gca aaa cgc att gca aac gaa aac caa      336
   Thr Arg Pro Tyr His Val Ile Ala Lys Arg Ile Ala Asn Glu Asn Gln
               100                 105                 110
   gcc agt ttc tgg gcg ttt gaa gaa ggc tat ttc cgc ccc tac tac atc      384
   Ala Ser Phe Trp Ala Phe Glu Glu Gly Tyr Phe Arg Pro Tyr Tyr Ile
           115                 120                 125
   acc tta gaa aaa gac ggc gtc aac gca ttt tcc ccg ttg ccg cgc cgt      432
   Thr Leu Glu Lys Asp Gly Val Asn Ala Phe Ser Pro Leu Pro Arg Arg
       130                 135                 140
   gcc gac ttt ttt ctt gaa caa ttc cct aag ctt gcc cag caa gaa tat      480
   Ala Asp Phe Phe Leu Glu Gln Phe Pro Lys Leu Ala Gln Gln Glu Tyr
   145                 150                 155                 160
   aaa gcg cca acg ccg gta cac ggc ggt ttt acg ccc atg gca aaa aac      528
   Lys Ala Pro Thr Pro Val His Gly Gly Phe Thr Pro Met Ala Lys Asn
                   165                 170                 175
   gct atc cgt tac tat atc gag ttg ttc cgc aat cta cgc aaa tac ccc      576
   Ala Ile Arg Tyr Tyr Ile Glu Leu Phe Arg Asn Leu Arg Lys Tyr Pro
               180                 185                 190
   gac tac atc cac cac cgc gca ccc aat gcc ggc cat tac ctc aaa ccg      624
   Asp Tyr Ile His His Arg Ala Pro Asn Ala Gly His Tyr Leu Lys Pro
           195                 200                 205
   tgg tcg ctc tcc atc ctc aag cgt ttg aac tac tat att gaa gac atc      672
   Trp Ser Leu Ser Ile Leu Lys Arg Leu Asn Tyr Tyr Ile Glu Asp Ile
       210                 215                 220
   caa atc gcc aaa cgt gtg gaa gca ggc aaa tac ggc aag ttt ttt att      720
   Gln Ile Ala Lys Arg Val Glu Ala Gly Lys Tyr Gly Lys Phe Phe Ile
   225                 230                 235                 240
   gtt ccc ttg cag gta ttc aac gac agc caa gtc cgt atc cat tgc gac      768
   Val Pro Leu Gln Val Phe Asn Asp Ser Gln Val Arg Ile His Cys Asp
                   245                 250                 255
   ttt ccc agc gtc cgc agc ttc ctg ctc cat gtt ttg agt tca ttt gcc      816
   Phe Pro Ser Val Arg Ser Phe Leu Leu His Val Leu Ser Ser Phe Ala
               260                 265                 270
   gaa cac gcg cct gcc gat acc aac atc atc atc aag cat cat cct atg      864
   Glu His Ala Pro Ala Asp Thr Asn Ile Ile Ile Lys His His Pro Met
           275                 280                 285
   gac cgc ggc ttt atc gac tac tgg cgc gac att aaa cgc ttt atc aaa      912
   Asp Arg Gly Phe Ile Asp Tyr Trp Arg Asp Ile Lys Arg Phe Ile Lys
       290                 295                 300
   gaa cac ccc gaa ctc aaa ggc cgt gtg att tat gtc cat gat gtc ccc      960
   Glu His Pro Glu Leu Lys Gly Arg Val Ile Tyr Val His Asp Val Pro
   305                 310                 315                 320
   ctg ccc gtt ttc ctg cgc cac ggt ctc ggc atg gtt acc atc aac agc     1008
   Leu Pro Val Phe Leu Arg His Gly Leu Gly Met Val Thr Ile Asn Ser
                   325                 330                 335
   acc agc ggc ctg tcc gga ctg att cac aat atg cca gtt aag gtt ctc     1056
   Thr Ser Gly Leu Ser Gly Leu Ile His Asn Met Pro Val Lys Val Leu
               340                 345                 350
   ggc cgt gcc tat tat gat att ccc ggc att act gac caa aat acc ttg     1104
   Gly Arg Ala Tyr Tyr Asp Ile Pro Gly Ile Thr Asp Gln Asn Thr Leu
           355                 360                 365
   gca gaa ttt tgg aat cat ccg aca ccg cct gac aaa gag ctg ttc cat     1152
   Ala Glu Phe Trp Asn His Pro Thr Pro Pro Asp Lys Glu Leu Phe His
       370                 375                 380
   gcc tac cga atg tac cac ctc aac gtg acc caa att aac ggc aac ttc     1200
   Ala Tyr Arg Met Tyr His Leu Asn Val Thr Gln Ile Asn Gly Asn Phe
   385                 390                 395                 400
   tac agt cag gtc ttt ttt cct aac aaa aac aca tcc gac tct tcc aca     1248
   Tyr Ser Gln Val Phe Phe Pro Asn Lys Asn Thr Ser Asp Ser Ser Thr
                   405                 410                 415
   cca aca acc                                                         1257
   Pro Thr Thr
<210> 8
   <211> 419
   <212> PRT
   <213> Neisseria meningitidis
<400> 8
   Met Lys Gln Thr Val Leu Lys Asn Asn Leu Gln Asn Leu Leu Glu Ser
   1 5 10 15
   Ala Glu Asn Ile Leu Leu Leu Gln Gly Pro Val Gly Asp Phe Phe Leu
               20                  25                  30
   Arg Leu Ala Asp Trp Leu Thr Ala Asn Gly Lys Thr Val His Lys Phe
           35                  40                  45
   Asn Phe Asn Ala Gly Asp Asp Tyr Phe Tyr Pro Pro Thr Gln Ala His
       50                  55                  60
   Thr Val Val Phe Asn Asp Asn Tyr Asp Ala Phe Pro Glu Phe Leu Gln
   65                  70                  75                  80
   Glu Tyr Ile Thr Gln His His Ile Gln Ala Val Val Cys Phe Gly Asp
                   85                  90                  95
   Thr Arg Pro Tyr His Val Ile Ala Lys Arg Ile Ala Asn Glu Asn Gln
               100                 105                 110
   Ala Ser Phe Trp Ala Phe Glu Glu Gly Tyr Phe Arg Pro Tyr Tyr Ile
           115                 120                 125
   Thr Leu Glu Lys Asp Gly Val Asn Ala Phe Ser Pro Leu Pro Arg Arg
       130                 135                 140
   Ala Asp Phe Phe Leu Glu Gln Phe Pro Lys Leu Ala Gln Gln Glu Tyr
   145                 150                 155                 160
   Lys Ala Pro Thr Pro Val His Gly Gly Phe Thr Pro Met Ala Lys Asn
                   165                 170                 175
   Ala Ile Arg Tyr Tyr Ile Glu Leu Phe Arg Asn Leu Arg Lys Tyr Pro
               180                 185                 190
   Asp Tyr Ile His His Arg Ala Pro Asn Ala Gly His Tyr Leu Lys Pro
           195                 200                 205
   Trp Ser Leu Ser Ile Leu Lys Arg Leu Asn Tyr Tyr Ile Glu Asp Ile
       210                 215                 220
   Gln Ile Ala Lys Arg Val Glu Ala Gly Lys Tyr Gly Lys Phe Phe Ile
   225                 230                 235                 240
   Val Pro Leu Gln Val Phe Asn Asp Ser Gln Val Arg Ile His Cys Asp
                   245                 250                 255
   Phe Pro Ser Val Arg Ser Phe Leu Leu His Val Leu Ser Ser Phe Ala
   260 265 270
   Glu His Ala Pro Ala Asp Thr Asn Ile Ile Ile Lys His His Pro Met
   275 280 285
   Asp Arg Gly Phe Ile Asp Tyr Trp Arg Asp Ile Lys Arg Phe Ile Lys
   290 295 300
   Glu His Pro Glu Leu Lys Gly Arg Val Ile Tyr Val His Asp Val Pro
   305 310 315 320
   Leu Pro Val Phe Leu Arg His Gly Leu Gly Met Val Thr Ile Asn Ser
   325 330 335
   Thr Ser Gly Leu Ser Gly Leu Ile His Asn Met Pro Val Lys Val Leu
   340 345 350
   Gly Arg Ala Tyr Tyr Asp Ile Pro Gly Ile Thr Asp Gln Asn Thr Leu
   355 360 365
   Ala Glu Phe Trp Asn His Pro Thr Pro Pro Asp Lys Glu Leu Phe His
   370 375 380
   Ala Tyr Arg Met Tyr His Leu Asn Val Thr Gln Ile Asn Gly Asn Phe
   385 390 395 400
   Tyr Ser Gln Val Phe Phe Pro Asn Lys Asn Thr Ser Asp Ser Ser Thr
   405 410 415
   Pro Thr Thr
<210> 9
   <211> 1008
   <212> DNA
   <213> Neisseria meningitidis
<220>
   <221> CDS
   <222> (1)..(1008)
<400> 9
   atg tcc atc aaa atc ctg att att tcc ccc agt tgg ata ggc gac tgc        48
   Met Ser Ile Lys Ile Leu Ile Ile Ser Pro Ser Trp Ile Gly Asp Cys
   1               5                   10                  15
   gtg atg acc cag ccc ttg ttc cgc cgt ttg aag gaa ctt cac ccc ggt        96
   Val Met Thr Gln Pro Leu Phe Arg Arg Leu Lys Glu Leu His Pro Gly
                   20                  25                  30
   tgc acg att gat gtg ttc gca ccg aag tgg tcg atg gcg gtg ttt gag       144
   Cys Thr Ile Asp Val Phe Ala Pro Lys Trp Ser Met Ala Val Phe Glu
           35                  40                  45
   cgt atg ccg gaa gtg aat gag att ctt gaa aat ccg ttc gga cac ggt       192
   Arg Met Pro Glu Val Asn Glu Ile Leu Glu Asn Pro Phe Gly His Gly
       50                  55                  60
   gcg ttg gag ttg aaa cgc cgt tgg cgg gtc ggt agg gat ttg ggg cgg       240
   Ala Leu Glu Leu Lys Arg Arg Trp Arg Val Gly Arg Asp Leu Gly Arg
   65                  70                  75                  80
   cgc gga tac gat cag gtt atc gtg ttg ccc ggt tct ttg aaa tcg gca       288
   Arg Gly Tyr Asp Gln Val Ile Val Leu Pro Gly Ser Leu Lys Ser Ala
                   85                  90                  95
   atc atc gcg ctg gcg aca ggt atc ggt aaa agg acg ggt tat gtc ggt       336
   Ile Ile Ala Leu Ala Thr Gly Ile Gly Lys Arg Thr Gly Tyr Val Gly
               100                 105                 110
   gaa agc cgt tat ttt ctg ttg aac gat ata cgc agg ctg gat aag gaa       384
   Glu Ser Arg Tyr Phe Leu Leu Asn Asp Ile Arg Arg Leu Asp Lys Glu
           115                 120                 125
   cgt ctg cct ttg atg gtg gac aga tat acg gct ctc gcg cat ccg agc       432
   Arg Leu Pro Leu Met Val Asp Arg Tyr Thr Ala Leu Ala His Pro Ser
       130                 135                 140
   cag gag gat ttt gac ggg cat tcg gga ttc ccc gag ttt tcc att gat       480
   Gln Glu Asp Phe Asp Gly His Ser Gly Phe Pro Glu Phe Ser Ile Asp
   145                 150                 155                 160
   gaa cgg cgg cgg gaa att tct gtc gaa acc ttt ggc ttg gat att gga       528
   Glu Arg Arg Arg Glu Ile Ser Val Glu Thr Phe Gly Leu Asp Ile Gly
                   165                 170                 175
   aag cct gtt ctg gct ttt tgt ccg ggt gcg gaa ttc ggg ccg gca aag       576
   Lys Pro Val Leu Ala Phe Cys Pro Gly Ala Glu Phe Gly Pro Ala Lys
               180                 185                 190
   cgt tgg ccg aca agg tat ttt gcc gag ttg ggc aaa cat tat tcg gcg       624
   Arg Trp Pro Thr Arg Tyr Phe Ala Glu Leu Gly Lys His Tyr Ser Ala
           195                 200                 205
   gcg ggt tgg cag gtt tgg ctg ttc ggt tcg caa aaa gat gat gaa att       672
   Ala Gly Trp Gln Val Trp Leu Phe Gly Ser Gln Lys Asp Asp Glu Ile
       210                 215                 220
   gcc gag gaa atc aac tgc ctt tca gac ggt atg tgt gtc aat ttg tgc       720
   Ala Glu Glu Ile Asn Cys Leu Ser Asp Gly Met Cys Val Asn Leu Cys
   225                 230                 235                 240
   ggc aaa acc gat ttg tcg cag gca atg gat ttg ctg tcg ttg gcg gac       768
   Gly Lys Thr Asp Leu Ser Gln Ala Met Asp Leu Leu Ser Leu Ala Asp
                   245                 250                 255
   acg gtc gtg tgc aac gac agc ggg ctg atg cac ttg gcg gcg gca ttg       816
   Thr Val Val Cys Asn Asp Ser Gly Leu Met His Leu Ala Ala Ala Leu
               260                 265                 270
   ggc agg aag gtg gtg gcg gtt tac ggt tct tcc agc ccg acg cat acg        864
   Gly Arg Lys Val Val Ala Val Tyr Gly Ser Ser Ser Pro Thr His Thr
           275                 280                 285
   ccg cct ttg agc gac agg gcg aaa atc gtc agc ctg cac ttg gaa tgt        912
   Pro Pro Leu Ser Asp Arg Ala Lys Ile Val Ser Leu His Leu Glu Cys
       290                 295                 300
   tcg ccc tgt ttt aag cgg gaa tgt ccg ctg ggg cat acc gac tgc ctc        960
   Ser Pro Cys Phe Lys Arg Glu Cys Pro Leu Gly His Thr Asp Cys Leu
   305                 310                 315                 320
   aac agg ctg tat ccc gag aag att gtg cag gcg gtt gaa gag gcg gta       1008
   Asn Arg Leu Tyr Pro Glu Lys Ile Val Gln Ala Val Glu Glu Ala Val
                   325                 330                 335
<210> 10
   <211> 336
   <212> PRT
   <213> Neisseria meningitidis
<400> 10
   Met Ser Ile Lys Ile Leu Ile Ile Ser Pro Ser Trp Ile Gly Asp Cys
   1               5                   10                  15
   Val Met Thr Gln Pro Leu Phe Arg Arg Leu Lys Glu Leu His Pro Gly
               20                  25                  30
   Cys Thr Ile Asp Val Phe Ala Pro Lys Trp Ser Met Ala Val Phe Glu
           35                  40                  45
   Arg Met Pro Glu Val Asn Glu Ile Leu Glu Asn Pro Phe Gly His Gly
       50                  55                  60
   Ala Leu Glu Leu Lys Arg Arg Trp Arg Val Gly Arg Asp Leu Gly Arg
   65                  70                  75                  80
   Arg Gly Tyr Asp Gln Val Ile Val Leu Pro Gly Ser Leu Lys Ser Ala
                   85                  90                  95
   Ile Ile Ala Leu Ala Thr Gly Ile Gly Lys Arg Thr Gly Tyr Val Gly
               100                 105                 110
   Glu Ser Arg Tyr Phe Leu Leu Asn Asp Ile Arg Arg Leu Asp Lys Glu
           115                 120                 125
   Arg Leu Pro Leu Met Val Asp Arg Tyr Thr Ala Leu Ala His Pro Ser
       130                 135                 140
   Gln Glu Asp Phe Asp Gly His Ser Gly Phe Pro Glu Phe Ser Ile Asp
   145 150 155 160
   Glu Arg Arg Arg Glu Ile Ser Val Glu Thr Phe Gly Leu Asp Ile Gly
   165 170 175
   Lys Pro Val Leu Ala Phe Cys Pro Gly Ala Glu Phe Gly Pro Ala Lys
   180 185 190
   Arg Trp Pro Thr Arg Tyr Phe Ala Glu Leu Gly Lys His Tyr Ser Ala
   195 200 205
   Ala Gly Trp Gln Val Trp Leu Phe Gly Ser Gln Lys Asp Asp Glu Ile
   210 215 220
   Ala Glu Glu Ile Asn Cys Leu Ser Asp Gly Met Cys Val Asn Leu Cys
   225 230 235 240
   Gly Lys Thr Asp Leu Ser Gln Ala Met Asp Leu Leu Ser Leu Ala Asp
   245 250 255
   Thr Val Val Cys Asn Asp Ser Gly Leu Met His Leu Ala Ala Ala Leu
   260 265 270
   Gly Arg Lys Val Val Ala Val Tyr Gly Ser Ser Ser Pro Thr His Thr
   275 280 285
   Pro Pro Leu Ser Asp Arg Ala Lys Ile Val Ser Leu His Leu Glu Cys
   290 295 300
   Ser Pro Cys Phe Lys Arg Glu Cys Pro Leu Gly His Thr Asp Cys Leu
   305 310 315 320
   Asn Arg Leu Tyr Pro Glu Lys Ile Val Gln Ala Val Glu Glu Ala Val
   325 330 335
<210> 11
   <211> 2277
   <212> DNA
   <213> Neisseria meningitidis
<220>
   <221> CDS
   <222> (1)..(2277)
<400> 11
   atg gca caa act aca ctc aaa ccc att gtt tta tca att ctt tta atc     48
   Met Ala Gln Thr Thr Leu Lys Pro Ile Val Leu Ser Ile Leu Leu Ile
   1               5                   10                  15
   aac aca ccc ctc ctc gcc caa gcg cat gaa act gag caa tcg gtg gat       96
   Asn Thr Pro Leu Leu Ala Gln Ala His Glu Thr Glu Gln Ser Val Asp
               20                  25                  30
   ttg gaa acg gtc agc gtc gtc ggc aaa agc cgt ccg cgc gcc acg tcg      144
   Leu Glu Thr Val Ser Val Val Gly Lys Ser Arg Pro Arg Ala Thr Ser
           35                  40                  45
   ggg ctg ttg cac act tcg acc gcc tcc gac aaa atc atc tcc ggc gat      192
   Gly Leu Leu His Thr Ser Thr Ala Ser Asp Lys Ile Ile Ser Gly Asp
       50                  55                  60
   acc ttg cgc caa aaa gcc gtc aac ttg ggc gac gct tta gac ggc gta      240
   Thr Leu Arg Gln Lys Ala Val Asn Leu Gly Asp Ala Leu Asp Gly Val
   65                  70                  75                  80
   ccg ggc atc cac gct tcg caa tac ggc ggc ggc gcg tct gct ccc gtc      288
   Pro Gly Ile His Ala Ser Gln Tyr Gly Gly Gly Ala Ser Ala Pro Val
                   85                  90                  95
   att cgc ggt caa aca ggc agg cgg att aaa gtg ttg aac cat cac ggc      336
   Ile Arg Gly Gln Thr Gly Arg Arg Ile Lys Val Leu Asn His His Gly
               100                 105                 110
   gaa aca ggc gat atg gcg gat ttt tcg ccc gat cac gcc att atg gta      384
   Glu Thr Gly Asp Met Ala Asp Phe Ser Pro Asp His Ala Ile Met Val
           115                 120                 125
   gat acc gcc ttg tcg caa cag gtc gaa atc ctg cgc ggg ccg gtt acg      432
   Asp Thr Ala Leu Ser Gln Gln Val Glu Ile Leu Arg Gly Pro Val Thr
       130                 135                 140
   ctc ttg tac agc tcg ggc aat gtg gcg ggg ctg gtc gat gtt gcc gat      480
   Leu Leu Tyr Ser Ser Gly Asn Val Ala Gly Leu Val Asp Val Ala Asp
   145                 150                 155                 160
   ggc aaa atc ccc gaa aaa atg cct gaa aac ggc gta tcg ggc gaa ctc      528
   Gly Lys Ile Pro Glu Lys Met Pro Glu Asn Gly Val Ser Gly Glu Leu
                   165                 170                 175
   gga ttg cgt ttg agc agc ggc aat ctg gaa aaa ctc acg tcc ggc ggc      576
   Gly Leu Arg Leu Ser Ser Gly Asn Leu Glu Lys Leu Thr Ser Gly Gly
               180                 185                 190
   atc aat atc ggt ttg ggc aaa aac ttt gta ttg cac acg gaa ggg ctg      624
   Ile Asn Ile Gly Leu Gly Lys Asn Phe Val Leu His Thr Glu Gly Leu
           195                 200                 205
   tac cgc aaa tcg ggg gat tac gcc gta ccg cgt tac cgc aat ctg aaa      672
   Tyr Arg Lys Ser Gly Asp Tyr Ala Val Pro Arg Tyr Arg Asn Leu Lys
       210                 215                 220
   cgc ctg ccc gac agc cac gcc gat tcg caa acg ggc agc atc ggg ctg      720
   Arg Leu Pro Asp Ser His Ala Asp Ser Gln Thr Gly Ser Ile Gly Leu
   225                 230                 235                 240
   tct tgg gtt ggc gaa aaa ggt ttt atc ggc gta gcg tac agc gac cgt      768
   Ser Trp Val Gly Glu Lys Gly Phe Ile Gly Val Ala Tyr Ser Asp Arg
                   245                 250                 255
   cgc gac caa tat ggt ctg cct gcc cac agc cac gaa tac gat gat tgc       816
   Arg Asp Gln Tyr Gly Leu Pro Ala His Ser His Glu Tyr Asp Asp Cys
               260                 265                 270
   cac gcc gac atc atc tgg caa aag agc ttg att aac aaa cgc tat tta       864
   His Ala Asp Ile Ile Trp Gln Lys Ser Leu Ile Asn Lys Arg Tyr Leu
           275                 280                 285
   cag ctt tat ccg cac ctg ttg acc gaa gaa gac atc gat tac gac aat       912
   Gln Leu Tyr Pro His Leu Leu Thr Glu Glu Asp Ile Asp Tyr Asp Asn
       290                 295                 300
   ccg ggc ttg agc tgc ggc ttc cac gac gac gat aat gca cac gca cac       960
   Pro Gly Leu Ser Cys Gly Phe His Asp Asp Asp Asn Ala His Ala His
   305                 310                 315                 320
   acc cac agc ggc aga ccg tgg ata gac ctg cgc aac aaa cgc tac gaa      1008
   Thr His Ser Gly Arg Pro Trp Ile Asp Leu Arg Asn Lys Arg Tyr Glu
                   325                 330                 335
   ctc cgt gcc gaa tgg aag caa ccg ttc ccc ggt ttt gaa gcc ctg cgc      1056
   Leu Arg Ala Glu Trp Lys Gln Pro Phe Pro Gly Phe Glu Ala Leu Arg
               340                 345                 350
   gta cac ctg aac cgc aac gac tac cgc cac gac gaa aaa gca ggc gat      1104
   Val His Leu Asn Arg Asn Asp Tyr Arg His Asp Glu Lys Ala Gly Asp
           355                 360                 365
   gca gtc gaa aac ttt ttt aac aac caa acg caa aac gcc cgc atc gag      1152
   Ala Val Glu Asn Phe Phe Asn Asn Gln Thr Gln Asn Ala Arg Ile Glu
       370                 375                 380
   ttg cgc cac caa ccc ata ggt cgt ctg aaa ggc agc tgg ggc gtg caa      1200
   Leu Arg His Gln Pro Ile Gly Arg Leu Lys Gly Ser Trp Gly Val Gln
   385                 390                 395                 400
   tat tta caa caa aaa tcc agt gct tta tct gcc ata tcc gaa gcg gtt      1248
   Tyr Leu Gln Gln Lys Ser Ser Ala Leu Ser Ala Ile Ser Glu Ala Val
                   405                 410                 415
   aaa caa ccg atg ctg ctt gac aac aaa gtg caa cat tac agc ttt ttc      1296
   Lys Gln Pro Met Leu Leu Asp Asn Lys Val Gln His Tyr Ser Phe Phe
               420                 425                 430
   ggt gta gaa cag gca aac tgg gac aac ttc acg ctt gaa gga ggc gta      1344
   Gly Val Glu Gln Ala Asn Trp Asp Asn Phe Thr Leu Glu Gly Gly Val
           435                 440                 445
   cgc gtg gaa aaa caa aaa gcc tcc att cag tac gac aaa gca ttg att      1392
   Arg Val Glu Lys Gln Lys Ala Ser Ile Gln Tyr Asp Lys Ala Leu Ile
       450                 455                 460
   gat cgg gaa aac tac tac aac cac ccc ctg ccc gac ctc ggc gcg cac      1440
   Asp Arg Glu Asn Tyr Tyr Asn His Pro Leu Pro Asp Leu Gly Ala His
   465                 470                 475                 480
   cgc caa acc gcc cgc tca ttc gca ctt tcg ggc aac tgg tat ttc acg      1488
   Arg Gln Thr Ala Arg Ser Phe Ala Leu Ser Gly Asn Trp Tyr Phe Thr
                   485                 490                 495
   cca caa cac aaa ctc agc ctg acc gcc tcc cat cag gaa cgc ctg ccg     1536
   Pro Gln His Lys Leu Ser Leu Thr Ala Ser His Gln Glu Arg Leu Pro
                   500                 505                 510
   tca acg caa gag ctg tac gca cac ggc aaa cac gtc gcc acc aac acc     1584
   Ser Thr Gln Glu Leu Tyr Ala His Gly Lys His Val Ala Thr Asn Thr
               515                 520                 525
   ttt gaa gtc ggc aac aaa cac ctc aac aaa gag cgt tcc aac aat atc     1632
   Phe Glu Val Gly Asn Lys His Leu Asn Lys Glu Arg Ser Asn Asn Ile
           530                 535                 540
   gaa ctc gcg ctg ggc tac gaa ggc gac cgc tgg caa tac aat ctg gca     1680
   Glu Leu Ala Leu Gly Tyr Glu Gly Asp Arg Trp Gln Tyr Asn Leu Ala
   545                 550                 555                 560
   ctc tac cgc aac cgc ttc ggt aac tac att tac gcc caa acc tta aac     1728
   Leu Tyr Arg Asn Arg Phe Gly Asn Tyr Ile Tyr Ala Gln Thr Leu Asn
                   565                 570                 575
   gac gga cgc ggc ccc aaa tcc atc gaa gac gac agc gaa atg aag ctc     1776
   Asp Gly Arg Gly Pro Lys Ser Ile Glu Asp Asp Ser Glu Met Lys Leu
               580                 585                 590
   gtg cgc tac aac caa tcc ggc gcc gac ttc tac ggc gcg gaa ggc gaa     1824
   Val Arg Tyr Asn Gln Ser Gly Ala Asp Phe Tyr Gly Ala Glu Gly Glu
           595                 600                 605
   atc tac ttc aaa ccg aca ccg cgc tac cgc atc ggc gtt tcc ggc gac     1872
   Ile Tyr Phe Lys Pro Thr Pro Arg Tyr Arg Ile Gly Val Ser Gly Asp
       610                 615                 620
   tat gta cga ggc cgt ctg aaa aac ctg cct tcc cta ccc ggc aga gaa     1920
   Tyr Val Arg Gly Arg Leu Lys Asn Leu Pro Ser Leu Pro Gly Arg Glu
   625                 630                 635                 640
   gat gcc tac ggc aac cgt cct ttc atc gca cag gac gac caa aat gcc     1968
   Asp Ala Tyr Gly Asn Arg Pro Phe Ile Ala Gln Asp Asp Gln Asn Ala
                   645                 650                 655
   ccc cgt gtt ccg gct gcg cgc ctc ggc ttc cac ctg aaa gcc tcg ctg     2016
   Pro Arg Val Pro Ala Ala Arg Leu Gly Phe His Leu Lys Ala Ser Leu
               660                 665                 670
   acc gac cgt atc gat gcc aat ttg gac tac tac cgc gtg ttc gcc caa     2064
   Thr Asp Arg Ile Asp Ala Asn Leu Asp Tyr Tyr Arg Val Phe Ala Gln
           675                 680                 685
   aac aaa ctc gcc cgc tac gaa acg cgc acg ccc gga cac cat atg ctc     2112
   Asn Lys Leu Ala Arg Tyr Glu Thr Arg Thr Pro Gly His His Met Leu
       690                 695                 700
   aac ctc ggc gca aac tac cgc cgc aat acg cgc tat ggc gag tgg aat     2160
   Asn Leu Gly Ala Asn Tyr Arg Arg Asn Thr Arg Tyr Gly Glu Trp Asn
   705                 710                 715                 720
   tgg tac gtc aaa gcc gac aac ctg ctc aac caa tcc gtt tac gcc cac     2208
   Trp Tyr Val Lys Ala Asp Asn Leu Leu Asn Gln Ser Val Tyr Ala His
                   725                 730                 735
   agc agc ttt ctc tct gat acg ccg caa atg ggc cgc agc ttt acc ggc     2256
   Ser Ser Phe Leu Ser Asp Thr Pro Gln Met Gly Arg Ser Phe Thr Gly
               740                 745                 750
   ggc gtg aac gtg aag ttt taa                                         2277
   Gly Val Asn Val Lys Phe
           755
<210> 12
   <211> 758
   <212> PRT
   <213> Neisseria meningitidis
<400> 12
   Met Ala Gln Thr Thr Leu Lys Pro Ile Val Leu Ser Ile Leu Leu Ile
   1 5 10 15
   Asn Thr Pro Leu Leu Ala Gln Ala His Glu Thr Glu Gln Ser Val Asp
   20 25 30
   Leu Glu Thr Val Ser Val Val Gly Lys Ser Arg Pro Arg Ala Thr Ser
   35 40 45
   Gly Leu Leu His Thr Ser Thr Ala Ser Asp Lys Ile Ile Ser Gly Asp
   50 55 60
   Thr Leu Arg Gln Lys Ala Val Asn Leu Gly Asp Ala Leu Asp Gly Val
   65 70 75 80
   Pro Gly Ile His Ala Ser Gln Tyr Gly Gly Gly Ala Ser Ala Pro Val
   85 90 95
   Ile Arg Gly Gln Thr Gly Arg Arg Ile Lys Val Leu Asn His His Gly
   100 105 110
   Glu Thr Gly Asp Met Ala Asp Phe Ser Pro Asp His Ala Ile Met Val
   115 120 125
   Asp Thr Ala Leu Ser Gln Gln Val Glu Ile Leu Arg Gly Pro Val Thr
   130 135 140
   Leu Leu Tyr Ser Ser Gly Asn Val Ala Gly Leu Val Asp Val Ala Asp
   145 150 155 160
   Gly Lys Ile Pro Glu Lys Met Pro Glu Asn Gly Val Ser Gly Glu Leu
   165 170 175
   Gly Leu Arg Leu Ser Ser Gly Asn Leu Glu Lys Leu Thr Ser Gly Gly
   180 185 190
   Ile Asn Ile Gly Leu Gly Lys Asn Phe Val Leu His Thr Glu Gly Leu
           195                 200                 205
   Tyr Arg Lys Ser Gly Asp Tyr Ala Val Pro Arg Tyr Arg Asn Leu Lys
       210                 215                 220
   Arg Leu Pro Asp Ser His Ala Asp Ser Gln Thr Gly Ser Ile Gly Leu
   225                 230                 235                 240
   Ser Trp Val Gly Glu Lys Gly Phe Ile Gly Val Ala Tyr Ser Asp Arg
                   245                 250                 255
   Arg Asp Gln Tyr Gly Leu Pro Ala His Ser His Glu Tyr Asp Asp Cys
               260                 265                 270
   His Ala Asp Ile Ile Trp Gln Lys Ser Leu Ile Asn Lys Arg Tyr Leu
           275                 280                 285
   Gln Leu Tyr Pro His Leu Leu Thr Glu Glu Asp Ile Asp Tyr Asp Asn
       290                 295                 300
   Pro Gly Leu Ser Cys Gly Phe His Asp Asp Asp Asn Ala His Ala His
   305                 310                 315                 320
   Thr His Ser Gly Arg Pro Trp Ile Asp Leu Arg Asn Lys Arg Tyr Glu
                   325                 330                 335
   Leu Arg Ala Glu Trp Lys Gln Pro Phe Pro Gly Phe Glu Ala Leu Arg
               340                 345                 350
   Val His Leu Asn Arg Asn Asp Tyr Arg His Asp Glu Lys Ala Gly Asp
           355                 360                 365
   Ala Val Glu Asn Phe Phe Asn Asn Gln Thr Gln Asn Ala Arg Ile Glu
       370                 375                 380
   Leu Arg His Gln Pro Ile Gly Arg Leu Lys Gly Ser Trp Gly Val Gln
   385                 390                 395                 400
   Tyr Leu Gln Gln Lys Ser Ser Ala Leu Ser Ala Ile Ser Glu Ala Val
                   405                 410                 415
   Lys Gln Pro Met Leu Leu Asp Asn Lys Val Gln His Tyr Ser Phe Phe
               420                 425                 430
   Gly Val Glu Gln Ala Asn Trp Asp Asn Phe Thr Leu Glu Gly Gly Val
           435                 440                 445
   Arg Val Glu Lys Gln Lys Ala Ser Ile Gln Tyr Asp Lys Ala Leu Ile
       450                 455                 460
   Asp Arg Glu Asn Tyr Tyr Asn His Pro Leu Pro Asp Leu Gly Ala His
   465                 470                 475                 480
   Arg Gln Thr Ala Arg Ser Phe Ala Leu Ser Gly Asn Trp Tyr Phe Thr
                   485                 490                 495
   Pro Gln His Lys Leu Ser Leu Thr Ala Ser His Gln Glu Arg Leu Pro
               500                 505                 510
   Ser Thr Gln Glu Leu Tyr Ala His Gly Lys His Val Ala Thr Asn Thr
           515                 520                 525
   Phe Glu Val Gly Asn Lys His Leu Asn Lys Glu Arg Ser Asn Asn Ile
       530                 535                 540
   Glu Leu Ala Leu Gly Tyr Glu Gly Asp Arg Trp Gln Tyr Asn Leu Ala
   545                 550                 555                 560
   Leu Tyr Arg Asn Arg Phe Gly Asn Tyr Ile Tyr Ala Gln Thr Leu Asn
                   565                 570                 575
   Asp Gly Arg Gly Pro Lys Ser Ile Glu Asp Asp Ser Glu Met Lys Leu
               580                 585                 590
   Val Arg Tyr Asn Gln Ser Gly Ala Asp Phe Tyr Gly Ala Glu Gly Glu
           595                 600                 605
   Ile Tyr Phe Lys Pro Thr Pro Arg Tyr Arg Ile Gly Val Ser Gly Asp
       610                 615                 620
   Tyr Val Arg Gly Arg Leu Lys Asn Leu Pro Ser Leu Pro Gly Arg Glu
   625                 630                 635                 640
   Asp Ala Tyr Gly Asn Arg Pro Phe Ile Ala Gln Asp Asp Gln Asn Ala
                   645                 650                 655
   Pro Arg Val Pro Ala Ala Arg Leu Gly Phe His Leu Lys Ala Ser Leu
               660                 665                 670
   Thr Asp Arg Ile Asp Ala Asn Leu Asp Tyr Tyr Arg Val Phe Ala Gln
           675                 680                 685
   Asn Lys Leu Ala Arg Tyr Glu Thr Arg Thr Pro Gly His His Met Leu
       690                 695                 700
   Asn Leu Gly Ala Asn Tyr Arg Arg Asn Thr Arg Tyr Gly Glu Trp Asn
   705                 710                 715                 720
   Trp Tyr Val Lys Ala Asp Asn Leu Leu Asn Gln Ser Val Tyr Ala His
                   725                 730                 735
   Ser Ser Phe Leu Ser Asp Thr Pro Gln Met Gly Arg Ser Phe Thr Gly
               740                 745                 750
   Gly Val Asn Val Lys Phe
           755
<210> 13
   <211> 1185
   <212> DNA
   <213> Neisseria meningitidis
<220>
   <221> CDS
   <222> (1)..(1185)
<400> 13
   atg aca gag gac gaa cgt ttc gcg tgg ctg caa ttg gcg ttt acg ccc       48
   Met Thr Glu Asp Glu Arg Phe Ala Trp Leu Gln Leu Ala Phe Thr Pro
   1               5                   10                  15
   tat atc ggc gcg gaa agt ttc ctg ctg ctg atg cgc cgt ttc ggc agc       96
   Tyr Ile Gly Ala Glu Ser Phe Leu Leu Leu Met Arg Arg Phe Gly Ser
               20                  25                  30
   gcg caa aat gcc ctg tcc gca ccg gcg gaa cag gtg gcg gca ctg ata      144
   Ala Gln Asn Ala Leu Ser Ala Pro Ala Glu Gln Val Ala Ala Leu Ile
           35                  40                  45
   cgg cac aaa cag gcg ctt gag gct tgg cgc aat gcg gaa aaa cgc gct      192
   Arg His Lys Gln Ala Leu Glu Ala Trp Arg Asn Ala Glu Lys Arg Ala
       50                  55                  60
   ctg gcg cgg cag gcg gca gaa gcg gcg ttg gaa tgg gaa atg cgg gac      240
   Leu Ala Arg Gln Ala Ala Glu Ala Ala Leu Glu Trp Glu Met Arg Asp
   65                  70                  75                  80
   gga tgc cgc ctg atg ctg ctt cag gat gaa gat ttc ccc gaa atg ctg      288
   Gly Cys Arg Leu Met Leu Leu Gln Asp Glu Asp Phe Pro Glu Met Leu
                   85                  90                  95
   acg cag ggg ctg acc gcg ccg ccg gtt ttg ttt ttg cgc ggc aac gtg      336
   Thr Gln Gly Leu Thr Ala Pro Pro Val Leu Phe Leu Arg Gly Asn Val
                   100                 105                 110
   cgg ctg ctg cac aag cct tcc gcc gcc atc gtc ggc agc cgc cat gcc      384
   Arg Leu Leu His Lys Pro Ser Ala Ala Ile Val Gly Ser Arg His Ala
           115                 120                 125
   acg ccg cag gcg atg cgg att gcc aaa gat ttc ggc aag tcg ttg ggc      432
   Thr Pro Gln Ala Met Arg Ile Ala Lys Asp Phe Gly Lys Ser Leu Gly
       130                 135                 140
   ggg caa aat atc ccc gtt gtg tcg ggt atg gct tcc ggt atc gat act      480
   Gly Gln Asn Ile Pro Val Val Ser Gly Met Ala Ser Gly Ile Asp Thr
   145                 150                 155                 160
   gcc gcc cat cag ggc gca ctg gag gca gaa ggc ggc acc atc gcc gtg      528
   Ala Ala His Gln Gly Ala Leu Glu Ala Glu Gly Gly Thr Ile Ala Val
                   165                 170                 175
   tgg ggg acg ggt ata gac cgc att tac ccg ccg tcc aat aaa aac ctt      576
   Trp Gly Thr Gly Ile Asp Arg Ile Tyr Pro Pro Ser Asn Lys Asn Leu
               180                 185                 190
   gcc tat gaa atc gcc gaa aaa gga ttg att gtc agc gag ttt ccc atc      624
   Ala Tyr Glu Ile Ala Glu Lys Gly Leu Ile Val Ser Glu Phe Pro Ile
           195                 200                 205
   ggt acg cgc ccg tat gcc ggc aat ttt ccg cgc cgc aac cgc ctg att      672
   Gly Thr Arg Pro Tyr Ala Gly Asn Phe Pro Arg Arg Asn Arg Leu Ile
       210                 215                 220
   gcc gcc ctg tcg caa gta acg ctg gtg gtt gaa gcc gcc ttg gaa tcc      720
   Ala Ala Leu Ser Gln Val Thr Leu Val Val Glu Ala Ala Leu Glu Ser
   225                 230                 235                 240
   ggt tcg ctg att acc gcc ggc ctg gcg gca gaa atg ggg cgc gaa gtg      768
   Gly Ser Leu Ile Thr Ala Gly Leu Ala Ala Glu Met Gly Arg Glu Val
                   245                 250                 255
   atg gcg gta ccc ggt tcg ata gac aat ccg cac agc aaa ggc tgc cac      816
   Met Ala Val Pro Gly Ser Ile Asp Asn Pro His Ser Lys Gly Cys His
               260                 265                 270
   aaa ctg att aaa gac ggc gca aaa ttg gtg gaa tgc ctg gac gac atc      864
   Lys Leu Ile Lys Asp Gly Ala Lys Leu Val Glu Cys Leu Asp Asp Ile
           275                 280                 285
   ctg aac gaa tgc ccg ggg cta ttg caa aat acg ggt gct tca tca tat      912
   Leu Asn Glu Cys Pro Gly Leu Leu Gln Asn Thr Gly Ala Ser Ser Tyr
       290                 295                 300
   tct ata aat aag gat acg ccc gac acg gga cgc cgc gcc gtt cag acg      960
   Ser Ile Asn Lys Asp Thr Pro Asp Thr Gly Arg Arg Ala Val Gln Thr
   305                 310                 315                 320
   gca tac gcc ccg ccg cct gcc gca aaa atg ccg tct gaa gcg gcg gca     1008
   Ala Tyr Ala Pro Pro Pro Ala Ala Lys Met Pro Ser Glu Ala Ala Ala
                   325                 330                 335
   ggc ggc aca gcc gtc ggc ggc ata ctg gat aaa atg ggt ttc gac ccc     1056
   Gly Gly Thr Ala Val Gly Gly Ile Leu Asp Lys Met Gly Phe Asp Pro
               340                 345                 350
   atc cat ccc gac gtg ctt gcc gga cag ttg gct atg cct gcc gca gat      1104
   Ile His Pro Asp Val Leu Ala Gly Gln Leu Ala Met Pro Ala Ala Asp
           355                 360                 365
   ttg tat gcc gca ctg ttg gaa ttg gaa ttg gac ggc agc gtt gcc gca      1152
   Leu Tyr Ala Ala Leu Leu Glu Leu Glu Leu Asp Gly Ser Val Ala Ala
       370                 375                 380
   atg ccc agc ggc aga tac cag cgt atc cga act                          1185
   Met Pro Ser Gly Arg Tyr Gln Arg Ile Arg Thr
   385                 390                 395
<210> 14
   <211> 395
   <212> PRT
   <213> Neisseria meningitidis
<400> 14
   Met Thr Glu Asp Glu Arg Phe Ala Trp Leu Gln Leu Ala Phe Thr Pro
   1               5                   10                  15
   Tyr Ile Gly Ala Glu Ser Phe Leu Leu Leu Met Arg Arg Phe Gly Ser
               20                  25                  30
   Ala Gln Asn Ala Leu Ser Ala Pro Ala Glu Gln Val Ala Ala Leu Ile
           35                  40                  45
   Arg His Lys Gln Ala Leu Glu Ala Trp Arg Asn Ala Glu Lys Arg Ala
       50                  55                  60
   Leu Ala Arg Gln Ala Ala Glu Ala Ala Leu Glu Trp Glu Met Arg Asp
   65                  70                  75                  80
   Gly Cys Arg Leu Met Leu Leu Gln Asp Glu Asp Phe Pro Glu Met Leu
                   85                  90                  95
   Thr Gln Gly Leu Thr Ala Pro Pro Val Leu Phe Leu Arg Gly Asn Val
               100                 105                 110
   Arg Leu Leu His Lys Pro Ser Ala Ala Ile Val Gly Ser Arg His Ala
           115                 120                 125
   Thr Pro Gln Ala Met Arg Ile Ala Lys Asp Phe Gly Lys Ser Leu Gly
       130                 135                 140
   Gly Gln Asn Ile Pro Val Val Ser Gly Met Ala Ser Gly Ile Asp Thr
   145                 150                 155                 160
   Ala Ala His Gln Gly Ala Leu Glu Ala Glu Gly Gly Thr Ile Ala Val
                   165                     170                 175
   Trp Gly Thr Gly Ile Asp Arg Ile Tyr Pro Pro Ser Asn Lys Asn Leu
               180                     185                 190
   Ala Tyr Glu Ile Ala Glu Lys Gly Leu Ile Val Ser Glu Phe Pro Ile
           195                     200                 205
   Gly Thr Arg Pro Tyr Ala Gly Asn Phe Pro Arg Arg Asn Arg Leu Ile
       210                     215                 220
   Ala Ala Leu Ser Gln Val Thr Leu Val Val Glu Ala Ala Leu Glu Ser
   225                     230                 235                 240
   Gly Ser Leu Ile Thr Ala Gly Leu Ala Ala Glu Met Gly Arg Glu Val
                   245                     250                 255
   Met Ala Val Pro Gly Ser Ile Asp Asn Pro His Ser Lys Gly Cys His
               260                     265                 270
   Lys Leu Ile Lys Asp Gly Ala Lys Leu Val Glu Cys Leu Asp Asp Ile
           275                     280                 285
   Leu Asn Glu Cys Pro Gly Leu Leu Gln Asn Thr Gly Ala Ser Ser Tyr
       290                     295                 300
   Ser Ile Asn Lys Asp Thr Pro Asp Thr Gly Arg Arg Ala Val Gln Thr
   305                     310                 315                 320
   Ala Tyr Ala Pro Pro Pro Ala Ala Lys Met Pro Ser Glu Ala Ala Ala
                   325                     330                 335
   Gly Gly Thr Ala Val Gly Gly Ile Leu Asp Lys Met Gly Phe Asp Pro
               340                     345                 350
   Ile His Pro Asp Val Leu Ala Gly Gln Leu Ala Met Pro Ala Ala Asp
           355                     360                 365
   Leu Tyr Ala Ala Leu Leu Glu Leu Glu Leu Asp Gly Ser Val Ala Ala
       370                     375                 380
   Met Pro Ser Gly Arg Tyr Gln Arg Ile Arg Thr
   385                     390                 395
<210> 15
<211> 24
   <212> DNA
   <213> Artificial
<220>
   <223> Primer Sequence
<400> 15
   ttggttaatt ggttgtaaca ctgg 24
<210> 16
   <211> 19
   <212> DNA
   <213> Artificial
<220>
   <223> Primer Sequence
<400> 16
   attctcatgt ttgacagcg 19
<210> 17
   <211> 18
   <212> DNA
   <213> Artificial
<220>
   <223> Primer Sequence
<400> 17
   atcctacaac ctcaagct 18
<210> 18
   <211> 18
   <212> DNA
   <213> Artificial
<220>
   <223> Primer Sequence
<400> 18
   atcccattct aaccaagc 18
<210> 19
   <211> 19
   <212> DNA
   <213> Artificial
<220>
   <223> Primer Sequence
<400> 19
   aagagattac gcgcagacc 19
<210> 20
   <211> 18
   <212> DNA
   <213> Artificial
<220>
   <223> Primer Sequence
<400> 20
   aatacgcaac cgcctctc 18

## Description

### Field of the Invention

This invention relates to virulence genes and proteins, and their use. More particularly, it relates to genes and proteins/peptides obtained from *Neisseria meningitidis,* and their use in therapy and in screening for drugs.

### Background of the Invention

*Neisseria meningitidis* is a Gram-negative bacterial pathogen that is implicated in septic shock and bacterial meningitis. This bacterium is a leading cause of bacterial meningitis in developed countries, and causes large-scale epidemics in Africa and China. In the UK, *Neisseria meningitidis* is the leading cause of death in childhood apart from road traffic accidents. The bacterium naturally inhabits the human naso-pharynx and then gains access to the blood stream from where it causes severe septicaemia or meningitis. Although current anti-microbials are effective in eliminating the bacterium from the body, the mortalilty from menigococcal septicaemia remains substantial. It would be desirable to provide means for treating or preventing conditions caused by *Neisseria meningitidis,* e.g. by Immunisation.

### Summary of the Invention

The present invention is based on the discovery of virulence genes in *Neisseria meningitidis.*

According to a first aspect of the invention, a peptide of the invention comprises the amino acid sequence identified herein as SEQ ID No. 2, 5 or a related molecule having at least 60% sequence similarity or identity at the peptide level, or an active fragment thereof that retains the ability to generate in an immune response an antibody that binds specifically to the full length of peptide, for therapeutic or diagnostic use.

The peptides may have many therapeutic uses for treating *Neisseria* infections, including use in vaccines for prophylactic application.

According to a second aspect, a polynucleotide encoding a peptide defined above, may also be useful for therapy or diagnosis.

According to a third aspect, the genes that encode the peptides may be utilised to prepare attenuated microorganisms. The attenuated microorganisms will usually have a mutation that disrupts the expression of one or more of the genes identified herein, to provide a strain that lacks virulence. These microorganisms will also have use in therapy and diagnosis.

According to a fourth aspect, the peptides, genes and attenuated microorganisms according to the invention may be used in the treatment or prevention of a condition associated with infection by *Neisseria* or Gram-negative bacteria.

### Description of the Invention

The present invention is based on the discovery of genes encoding peptides which are implicated in virulence. The peptides and genes of the invention are therefore useful for the preparation of therapeutic agents to treat Infection. It should be understood that references to therapy also include preventative treatments, e.g. vaccination. Furthermore, while the products of the invention are intended primarily for treatment of infections in human patients, veterinary applications are also considered to be within the scope of the invention.

The present invention is described with reference to *Neisseria meningitidis.* However, all the *Neisseria* strains, and many other Gram-negative bacterial strains are likely to include related peptides or proteins having amino acid sequence identity or similarity to those identified herein. Organisms likely to contain the peptides include, but are not limited to the genera *Salmonella, Enterobacter, Klebsiella, Shigella* and *Yersinia.*

The experiments carried out to identify the virulence genes of the invention utilised *N. meningitidis* strain B. Homology searches were performed on the strain A database, however the proteins and genes from strain B are preferred.

Preferably, the peptides that may be useful in the various aspects of the invention have greater than 60% similarity or identity with the peptides identified herein. Most preferably, the peptides have greater than 80% sequence similarity or identity, e.g. 95% similarity.

The terms "similarity" and "identity" are known in the art. The use of the term "identity" refers to a sequence comparison based on identical matches between correspondingly identical positions in the sequences being compared. The term. "similarity" refers to a comparison between amino acid sequences, and takes into account not only identical amino acids in corresponding positions, but also functionally similar amino acids in corresponding positions. Thus similarity between polypeptide sequences indicates functional similarity, in addition to sequence similarity.

Levels of identity between gene sequences and levels of identity or similarity between amino acid sequences can be calculated using known methods. In relation to the present invention, publicly available computer based methods for determining identity and similarity include the BLASTP, BLASTN and FASTA (Atschul et al., J. Molec. Biol., 1990; 215:403-410), the BLASTX program available from NCBI, and the Gap program from Genetics Computer Group, Madison WI. The levels of similarity and identity provided herein, were obtained using the Gap program, with a Gap penalty of 12 and a Gap length penalty of 4 for determining the amino acid sequence comparisons, and a Gap penalty of 50 and a Gap length penalty of 3 for the polynucleotide sequence comparisons.

Having characterised a gene according to the invention, it is possible to use the gene sequence to search for related genes or peptides in other microorganisms. This may be carried out by searching in existing databases, e.g. EMBL or GenBank.

Peptides or proteins according to the invention may be purified and isolated by methods known in the art. In particular, having identified the gene sequence, it will be possible to use recombinant techniques to express the genes in a suitable host. Active fragments and related molecules can be identified and may be useful in therapy. For example, the peptides or their active fragments may be used as antigenic determinants in a vaccine, to elicit an immune response. They may also be used in the preparation of antibodies, for passive immunisation, or diagnostic applications. Suitable antibodies include monoclonal antibodies, or fragments thereof, including single chain Fv fragments. Methods for the preparation of antibodies will be apparent to those skilled in the art.

Active fragments of the peptides are those that retain the biological function of the peptide. For example, when used to elicit an immune response, the fragment will be of sufficient size, such that antibodies generated from the fragment will discriminate between that peptide and other peptides on the bacterial microorganism. Typically, the fragment will be at least 30 nucleotides (10 amino acids) in size, preferably 60 nucleotides (20 amino acids) and most preferably greater than 90 nucleotides (30 amino acids) in size.

It should also be understood, that in addition to related molecules from other microorganisms, the invention encompasses modifications made to the peptides and polynucleotides identified herein which do not significantly alter the biological function. It will be apparent to the skilled person that the degeneracy of the genetic code can result in polynucleotides with minor base changes from those specified herein, but which nevertheless encode the same peptides. Complementary polynucleotides are also within the invention. Conservative replacements at the amino acid level are also envisaged, i.e. different acidic or basic amino acids may be substituted without substantial loss of function.

The preparation of vaccines based on attenuated microorganisms is known to those skilled in the art. Vaccine compositions can be formulated with suitable carriers or adjuvants, e.g. alum, as necessary or desired, to provide effective immunisation against infection. The preparation of vaccine formulations will be apparent to the skilled person. The attenuated microorganisms may be prepared with a mutation that disrupts the expression of any of the genes identified herein. The skilled person will be aware of methods for disrupting expression of particular genes. Techniques that may be used include insertional inactivation or gene deletion techniques. Attenuated microorganisms according to the invention may also comprise additional mutations in other genes, for example in a second gene identified herein or in a separate gene required for growth of the microorganism, e.g. an *aro* mutation or, with regard to *Salmonella,* in a gene located in the SPI2 region identified in WO-A-96/17951.

Attenuated microorganisms may also be used as carrier systems for the delivery of heterologous antigens, therapeutic proteins or nucleic acids (DNA or RNA). In this embodiment, the attenuated microorganisms are used to deliver a heterologous antigen, protein or nucleic acid to a particular site *in vivo.* Introduction of a heterologous antigen, peptide or nucleic acid into an attenuated microorganism can be carried out by conventional techniques, including the use of recombinant constructs, e.g. vectors, which comprise polynucleotides that express the heterologous antigen or therapeutic protein, and also include suitable promoter sequences. Alternatively, the gene that encodes the heterologous antigen or protein may be incorporated into the genome of the organism and the endogenous promoters used to control expression.

More generally, and as is well known to those skilled in the art, a suitable amount of an active component of the invention can be selected, for therapeutic use, as can suitable carriers or excipients, and routes of administration. These factors would be chosen or determined according to known criteria such as the nature/severity of the condition to be treated, the type and/or health of the subject etc.

In a separate embodiment, the products of the invention may be used in screening assays for the identification of potential antimicrobial drugs or for the detection for virulence. Routine screening assays are known to those skilled in the art, and can be adapted using the products of the invention in the appropriate way. For example, the products of the invention may be used as the target for a potential drug, with the ability of the drug to inactivate or bind to the target indicating its potential antimicrobial activity.

The various products of the invention may also be used in veterinary applications.

The following is a brief overview of the experimental procedure used to identify the virulence genes.

Signature-tagged mutagenesis (STM) (Hensel et al., Science, 1995; 269: 400-403) was used to identify genes in *N. meningitidis* that are essential for septicemic infection. In STM, individual mutants are tagged with unique sequence identifiers, allowing large numbers of mutants to be analyzed simultaneously. However, it is necessary to construct libraries of insertional mutants, so far a limitation in studying *N. meningitidis.* Mutagenesis was accomplished successfully using a method in which *Neisseria* DNA is modified *in vitro* using purified components of Tn 10 transposition. As *N. meningitidis* efficiently takes up exogenous DNA, the modified alleles are then introduced into *N. meningitidis* by transformation. The mutants can then be screened for their ability to cause systemic infection.

The vector pSTM115 (Sun et al., Nature Medicine, 2000; 6(11): 1269-1273) was used as the transposon donor for *in vitro* mutagenesis. 96 pSTM115 derivatives, each containing unique signature tags, were included in 96 separate transposition reactions. The modified genomic DNA was repaired, and returned to the host by transformation. To determine whether Tn10 insertion occurs at diverse sites, 40 transformants were assessed from a single transposition reaction by Southern blot analysis. Each had a single, distinct Tn 10 insertion. To establish whether Tn 10 integration was stable during systemic infection of infant rats, the hydridization patterns of six mutants before and after passage through rats were compared. Identical hybridization patterns before and after infection were obtained.

The experimental conditions used were as follows:

### Bacterial strains and growth:

C311+ is an ET-5, serogroup B *N. meningitidis* isolate from a patient with invasive meningococcal infection (Virji et al., Mol. Microbiol., 1991; 5: 1831-1841). *N. meningitidis* was grown on brain-heart infusion medium with 5% Levinthal's supplement. *E. coli* strains were propagated on Luria Bertani media. Kanamycin was added to solid media as required at concentrations of 75 and 50 µg/ml for *N. meningitidis* and *E. coli,* respectively.

### In vitro transposition and insertion site characterization:

The pACYC184 origin of replication in pSTM115 was used to isolate the insertion site by marker rescue. Nucleotide sequencing was carried out using the dye-termination method (Perkin Elmer, Norwalk, Connecticut) with primers NG62 (5'-TTGGTTAATTGGTTGTAACACTGG-3') (SEQ ID NO. 15) or NG99 (5'-ATTCTCATGTTTGACAGCG-3') (SEQ ID NO. 16). Homology searches were performed against protein databases (http://www.ncbi.nlm.nih-gov/), including the serogroup A and B *N.meningitidis* and the *N. gonorrhoeae* genome sequences (http://www.sanger.ac.uk/Projects/N_meningitidis and http://www.tigr.org, and http://dna1.chem.ou.edu/gono.html, respectively).

### Tag amplification, cloning and Southern blot analyses:

Hybridizations and preparations of dot blots were performed as described in Hensel *et al., supra,* except that tags were amplified with primers NG13 (5'-ATCCTACAACCTCAAGCT-3') (SEQ ID NO. 17) and NG14 (5'-ATCCCATTCTAACCAAGC-3') (SEQ ID NO. 18), and PCR products, rather than plasmid DNA, were fixed onto membranes. Oilgonucleotides S1 (5'-AAGAGATTACGCGCAGACC-3') (SEQ ID NO. 19) and S2 (5'-AATACGCAACCGCCTCTC-3') (SEQ ID NO. 20) anneal to sequences in pSTM115 flanking the 'signature tags' and were used to amplify a 367-base-pair product from each pSTM115 derivative. For Southern blot analysis, the kanamycin-resistance cassette from pSTM 115 was labelled using the random primers method (NEB), and was used as a probe against genomic DNA digested with *Cla*I*.*

### Animal model:

For screening the STM pools, mutants were grown individually for 18 h in microtiter plates. The bacteria were pooled, then re-suspended in PBS. Wistar rats (5 days old) were inoculated intraperitoneally with 100 µl of the suspension, and were monitored for 48 h. To establish the competitive index of a mutant, wild-type and mutant bacteria were grown for 18 h on solid media and collected into PBS, and rats were inoculated with a 1:1 ratio of mutant to wild-type cells in a total inoculum of 5 x 10⁸ CFU. The proportion of mutant (kanamycin-resistant) to wild-type (kanamycin-sensitive) bacteria was determined by plating replicate samples to media with or without added antibiotic.

The results of the homology searches are shown in Table 1.

**Table 1**

| SEQ ID NO. | PROTEIN | ACCESSION NO. | ORGANISM |
|---|---|---|---|
| 1 & 2 | Polyribonucleotide nucleotidyl transferase | NMB0758 | N. meningitidis |

The gene products were used to produce polyclonal antibodies that were tested in an Elisa assay against various *N. meningitidis* strains to evaluate their effectiveness as a vaccine candidate. The strains used were:
*Neisseria meningitidis* (B) Type 1000
*Neisseria meningitidis* (B) Type NGE31
*Neisseria meningitidis* (B) Type NGH15
*Neisseria meningitidis* (B) Type SW2 107
*Neisseria meningitidis* (B) Type NHG38
*Neisseria meningitidis* (B) Type NGE28
*Neisseria meningitidis* (B) Type 2996

This provides information as to the variety of *N. meningitidis* strains that are recognised by these antibodies.

*N. Meningitidis* was grown on Columbia agar with chocolated horse blood (Oxoid) for 14 hours at 37°C in 5% CO₂. The cells were scraped from agar plates and resuspended in 20ml PBS in a 50ml tube. The cell suspension was heated for 30 minutes at 56°C to kill the bacteria.

A 50 µl sample of the heat-killed *N*. *Meningitidis* was spread on the Columbia agar with the chocolated horse blood and incubated for 18 hours at 37C, 5%CO₂. This allows confirmation that all *N. Meningitidis* cells have been killed. The OD₆₂₀ of the suspension is adjusted to 0.1 OD units versus PBS. Elisa with heat killed *N. meningitidis*

Elisa assays were carried out using the heat-killed *N. meningitidis* using the following protocol. Elisa plates were coated overnight with heat-killed cells (50µl of killed bacteria in PBS to each well of 96 well plate and incubated 4°C).

Standard Elisa protocols were followed, with all incubations at 37°C for 1 hour. PBS/3% BSA blocking solution, PBS/Tween 0.1% wash solution, anti-rabbit AP conjugate secondary antibody (Sigma) and Sigma fast P Nitrophenyl phosphate detection reagent (Sigma) were utilised. The data was read at 405nm using an appropriate micro-titre plate reader. The sera used was that available seven days after the first booster vaccination (day 35 after first vaccination).

The antibodies tested were those raised against the gene products identified as SEQ ID NOS. 4, 6, 8, 10 and 12. In each case, the results showed that the antisera recognised several different strains of *N*. *meningitidis B.*

### Ex vivo/in vitro screening.

Protection against meningococcal disease in humans has been associated with the presence of bacteriocidal antibodies against *N. meningitidis* (Goldscheider et al. J. Exp. Med., 1969; 129: 1307-1326). There is also evidence to suggest a correlation between the presence of detectable bacteriocidal activity and protection in an *in vivo* model (reference Martin, J. Bacteriol., 2000; 83: 27-31). Therefore, the antiserum generated was used to evaluate the bacteriocidal activity of the antibodies generated.

The bactericidal assays were performed with pre-immune sera and the corresponding rabbit antiserum raised against the candidate antigens. Commercially available rabbit serum was used as the complement source following pre-screening to eliminate complement only killing. Dulbecoo's PBS (Gibco) was used as a buffer where necessary. The *N. meningitidis* strain MC58, was grown at 37°C (5% CO₂) for 14 hours prior to use in the assay.

200-400 CFU of MC58 in a 50µl volume were incubated in the presence of complement (50µl) with 100µl of serial dilutions of heat-inactivated serum. Samples at time zero were plated to Columbia agar with chocolated horse blood (Oxoid). After incubation for 60 minutes, the number of surviving bacteria was evaluated by plating to Columbia agar with chocolated horse blood (Oxoid). The bacteriocidal activity was expressed in terms of percentage of bacteria surviving after 60 minutes. All samples were tested in duplicate and plated in triplicate. All appropriate positive and negative controls were utilised. In each test sample, the bacteriocidal activity was substantially greater than that for the pre-immune sera.

### In vivo screening.

To evaluate the protective efficacy of vaccine candidates, adult mice were immunised with the recombinant proteins identified herein as SEQ ID NOS. 6 and 14 and the protective response determined by live bacterial challenge. For each vaccine candidate 15 six week old mice (6 week old balb/C mice) were vaccinated (subcutaneously) with 25µg of antigen on two separate occasions at three week intervals.

One week after the end of the immunisation schedule, the group was challenged with the homologous bacterial strain MC58. The bacteria were inoculated intraperiponeally in a volume of 500µl in Brain Heart Infusion/ 0.5% iron dextran media at a dose of 10⁷ cfu. Previous results have shown that iron is required for initiation of bacteraemic disease in these animals. This model has previously been used to demonstrate the protective efficacy of vaccination (Lissolo et al., Infect. Immujn., 1995; 63: 884-890).

Control groups included animals vaccinated with adjuvant alone (negative control) or with adjuvant combined with purified PorA (positive control). PorA is an outer membrane protein expressed exclusively by *N. meningitidis* and is the principal target for bactericidal antibodies induced by outer membrane vesicle vaccines. Monoclonal antibodies against PorA have also been shown to passively protect animals in the Infant rat model. PorA however varies considerably between strains and so while it elicits some protection when challenged with a homologous strain, it is not an ideal vaccine candidate. Survival was monitored following challenge. The negative control showed no survival after 48 hours. Those vaccinated with PorA showed 6 survivors at 72 hours. Those vaccinated with the proteins of SEQ ID NOS. 6 and 14 showed 5 and 3 survivors, respectively.

## Claims

1. A peptide comprising the amino acid sequence identified herein as SEQ ID NO. 2, or a related molecule having at least 60% sequence similarity or identity at the peptide level, or an active fragment thereof that retains the ability to generate in an immune response an antibody that binds specifically to the full length of peptide, for therapeutic or diagnostic use.

2. A peptide according to claim 1, wherein the sequence similarity or identity is at least 80%.

3. A peptide according to claim 1, wherein the sequence similarity or identity is at least 90%.

4. A peptide according to claim 1, comprising the amio acid sequence identified herein as SEQ ID NO. 2.

5. A polynucleotide encoding a peptide according to any preceding claim for therapeutic or diagnostic use.

6. An attenuated microorganism comprising a mutation that disrupts the expression of the nucleotide sequence defined as SEQ ID NO. 1.

7. A microorganism according to claim 6, wherein the mutation is insertional inactivation or a gene deletion.

8. A microorganism according to claim 6 or claim 7, wherein the microorganism is *Neisseria meningitidis.*

9. A microorganism according to any of claims 6 to 8, comprising a second mutation in a second nucleotide sequence.

10. A microorganism according to any of claims 6 to 9, for therapeutic or diagnostic use.

11. A microorganism according to any of claims 6 to 10, comprising a heterologous antigen, therapeutic peptide or nucleic acid.

12. A vaccine comprising a peptide according to any of claims 1 to 4, or the means for its expression.

13. A vaccine comprising a microorganism according to any of claims 6 to 11.

14. Use of a product according to any of claims 1 to 11 for the manufacture of a medicament for use in the treatment or prevention of a condition associated with infection by *Neisseria* or Gram-negative bacteria.

15. Use according to claim 14, wherein the condition is meningitis.

16. Use according to claim 14 or claim 15, for veterinary treatment.

17. Use of a product according to any of claims 1 to 11, in a screening assay for the identification of an antimicrobial drug.

## Patentansprüche

1. Peptid, das die vorliegend mit SEQ ID NO: 2 bezeichnete Aminosäuresequenz umfasst, oder ein verwandtes Molekül mit einer Sequenzähnlichkeit oder -identität auf Peptidebene von mindestens 60%, oder ein wirksames Fragment davon, das die Befähigung zur Erzeugung eines Antikörpers, der das vollständige Peptid spezifisch bindet, in einer Immunantwort beibehält, zur therapeutischen Verwendung.

2. Peptid nach Anspruch 1, wobei die Sequenzähnlichkeit oder -identität mindestens 80% beträgt.

3. Peptid nach Anspruch 1, wobei die Sequenzähnlichkeit oder -identität mindestens 90% beträgt.

4. Peptid nach Anspruch 1, dass die vorliegend mit SEQ ID NO: 2 bezeichnete Aminosäuresequenz umfasst.

5. Polynukleotid zur therapeutischen Verwendung, dass für ein Peptid nach einem der vorhergenden Ansprüche kodiert.

6. Abgeschwächter Mikroorganismus, der eine Mutation aufweist, welche die Expression der in der SEQ ID NO: 1 definierten Nukleotidsequenz aufhebt.

7. Mikroorganismus nach Anspruch 6, wobei die Mutation eine Insertionsinaktivierung oder Gendeletion ist.

8. Mikroorganismus nach Anspruch 6 oder 7, wobei der Mikroorganismus *Neisseria meningitidis* ist.

9. Mikroorganismus nach einem der Ansprüche 6 bis 8, der eine zweite Mutation in einer zweiten Nukleotidsequenz aufweist.

10. Mikroorganismus nach einem der Ansprüche 6 bis 9 zur therapeutischen oder diagnostischen Verwendung.

11. Mikroorganismus nach einem der Ansprüche 6 bis 10, der ein heterologes Antigen, therapeutisches Peptid oder Nukleinsäure umfasst.

12. Vakzine, die ein Peptid nach einem der Ansprüche 1 bis 4 oder das Mittel zu dessen Expression umfasst.

13. Vakzine, die einen Mikroorganismus nach einem der Ansprüche 6 bis 11 umfasst.

14. Verwendung eines Produkts nach einem der Ansprüche 1 bis 11 zur Herstellung eines Medikaments zur Verwendung in der Behandlung oder Prävention eines mit einer Infektion mit *Neisseria* oder Gram-negativen Bakterien zusammenhängenden Zustands.

15. Verwendung nach Anspruch 14, wobei der Zustand eine Meningitis ist.

16. Verwendung nach Anspruch 14 oder 15 zur veterinärmedizinischen Behandlung.

17. Verwendung eines Produkts nach einem der Ansprüche 1 bis 11 in einem Screeningtest zur Identifizierung eines antimikrobiellen Wirkstoffs.

## Revendications

1. Peptide comprenant la séquence d'aminoacides identifiée ici comme étant SEQ ID NO. 2, ou une molécule apparentée ayant une similarité ou identité de séquence d'au moins 60% au niveau peptidique, ou un fragment actif de celui-ci qui conserve l'aptitude à produire, dans une réponse immunitaire, un anticorps qui se lie spécifiquement à la longueur totale du peptide, destiné à une utilisation thérapeutique ou diagnostique.

2. Peptide selon la revendication 1, où la similarité ou identité de séquence est d'au moins 80%.

3. Peptide selon la revendication 1, où la similarité ou identité de séquence est d'au moins 90%.

4. Peptide selon la revendication 1, comprenant la séquence d'aminoacides identifiée ici comme étant SEQ ID NO. 2.

5. Polynucléotide codant un peptide selon l'une quelconque des revendications précédentes destiné à une utilisation thérapeutique ou diagnostique.

6. Microorganisme atténué comprenant une mutation qui interrompt l'expression de la séquence nucléotidique définie comme étant SEQ ID NO. 1.

7. Microorganisme selon la revendication 6 où la mutation est une inactivation insertionnelle ou une délétion génique.

8. Microorganisme selon la revendication 6 ou la revendication 7, où le microorganisme est *Neisseria meningitidis.*

9. Microorganisme selon l'une quelconque des revendications 6 à 8, comprenant une seconde mutation dans une seconde séquence nucléotidique.

10. Microorganisme selon l'une quelconque des revendications 6 à 9, destiné à une utilisation thérapeutique ou diagnostique.

11. Microorganisme selon l'une quelconque des revendications 6 à 10, comprenant un antigène hétérologue, un peptide thérapeutique ou un acide nucléique.

12. Vaccin comprenant un peptide selon l'une quelconque des revendications 1 à 4, ou les moyens pour son expression.

13. Vaccin comprenant un microorganisme selon l'une quelconque des revendications 6 à 11.

14. Utilisation d'un produit selon l'une quelconque des revendications 1 à 11 pour la fabrication d'un médicament destiné à être utilisé dans le traitement ou la prévention d'une affection associée avec une infection par *Neisseria* ou des bactéries Gram-négatives.

15. Utilisation selon la revendication 14, où l'affection est la méningite.

16. Utilisation selon la revendication 14 ou la revendication 15, destinée à un traitement vétérinaire.

17. Utilisation d'un produit selon l'une quelconque des revendications 1 à 11, dans un essai de criblage pour l'identification d'un médicament antimicrobien.
